(19) **Europäisches Patentamt / European Patent Office / Office européen des brevets**

(11) **EP 4 458 864 A1**

(12) # EUROPEAN PATENT APPLICATION
published in accordance with Art. 153(4) EPC

(43) Date of publication:
**06.11.2024 Bulletin 2024/45**

(21) Application number: **23735114.3**

(22) Date of filing: **03.01.2023**

(51) International Patent Classification (IPC):
**C07K 19/00** (2006.01)　　**C12N 15/00** (2006.01)
**A61P 31/00** (2006.01)

(52) Cooperative Patent Classification (CPC):
**A01N 37/46; A01P 1/00; A61K 8/64; A61K 9/00;**
**A61K 38/16; A61K 47/32; A61K 47/36;**
**A61K 47/38; A61K 47/42; A61P 31/00;**
**A61P 31/14; A61Q 17/00; C07K 19/00;**
**C12N 15/00;** Y02A 50/30

(86) International application number:
**PCT/CN2023/070015**

(87) International publication number:
**WO 2023/126009 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 31.12.2021　CN 202111668011
　　　　　　30.01.2022　CN 202210114482
　　　　　　01.07.2022　CN 202210773393
　　　　　　12.08.2022　CN 202210968998

(71) Applicant: **Kangma-Healthcode (Shanghai) Biotech Co., Ltd**
**Pudong New Area**
**Shanghai 201321 (CN)**

(72) Inventors:
- **GUO, Min**
  **Shanghai 201321 (CN)**
- **XU, Liqiong**
  **Shanghai 201321 (CN)**
- **LIU, Zhang**
  **Shanghai 201321 (CN)**
- **ZHANG, Jun**
  **Shanghai 201321 (CN)**
- **WU, Zhi**
  **Shanghai 201321 (CN)**
- **YU, Xue**
  **Shanghai 201321 (CN)**

(74) Representative: **Murgitroyd & Company**
**165-169 Scotland Street**
**Glasgow G5 8PL (GB)**

(54) **POLYMER MOLECULE, MONOMERIC STRUCTURE AND POLYMERIC STRUCTURE COMPRISING SAME**

(57)　The present invention provides a polymer molecule, a monomeric structure and a polymeric structure comprising same, a related product, a preparation method, and uses. A polymer blocking the binding to a receptor is formed by means of the polymerization effect of the polymer molecule, thus effectively increasing the binding capability with respect to a virus. The polymer molecule is characterized in that: multiple binding-blocking molecular units used for blocking the binding between the virus and a cell receptor are polymerized into the polymeric structure.

Figure 24

# SEQUENCE LISTING

<110> KANGMA-HEATHCODE (SHANGHAI) BIOTECH CO., LTD.

<120> POLYMER MOLECULE, MONOMERIC STRUCTURE AND POLYMER STRUCTURE COMPRISING SAME

<130> 2022

<160> 19

<170> PatentIn version 3.3

<210> 1

<211> 140

<212> PRT

<213> artificial sequence

<400> 1

Ser Asp Val Gln Ser Ser Leu Thr Gly Thr Trp Tyr Asn Glu Leu Asn
1               5                   10                  15

Ser Lys Met Glu Leu Thr Ala Asn Lys Asp Gly Thr Leu Thr Gly Lys
                20                  25                  30

Tyr Leu Ser Lys Val Gly Asp Val Tyr Val Pro Tyr Pro Leu Ser Gly
                35                  40                  45

Arg Tyr Asn Leu Gln Pro Pro Ala Gly Gln Gly Val Ala Leu Gly Trp

50            55           60

Ala Val Ser Trp Glu Asn Ser Lys Ile His Ser Ala Thr Thr Trp Ser

65           70           75           80

Gly Gln Phe Phe Ser Glu Ser Ser Pro Val Ile Leu Thr Gln Trp Leu

85           90           95

Leu Ser Ser Ser Thr Ala Arg Gly Asp Val Trp Glu Ser Thr Leu Val

100           105           110

Gly Asn Asp Ser Phe Thr Lys Thr Ala Pro Thr Glu Gln Gln Ile Ala

115           120           125

His Ala Gln Leu His Cys Arg Ala Pro Arg Leu Lys

130           135           140

<210> 2

<211> 238

<212> PRT

<213> artificial sequence

<400> 2

Val Ser Lys Gly Glu Glu Leu Phe Thr Gly Val Val Pro Ile Leu Val

1　　　　　　5　　　　　　　　　10　　　　　　　　15

Glu Leu Asp Gly Asp Val Asn Gly His Lys Phe Ser Val Arg Gly Glu

　　　　　　20　　　　　　　25　　　　　　　30

Gly Glu Gly Asp Ala Thr Asn Gly Lys Leu Thr Leu Lys Phe Ile Cys

　　　35　　　　　　　40　　　　　　　45

Thr Thr Gly Lys Leu Pro Val Pro Trp Pro Thr Leu Val Thr Thr Leu

　　50　　　　　　　55　　　　　　　60

Thr Tyr Gly Val Gln Cys Phe Ser Arg Tyr Pro Asp His Met Lys Gln

65　　　　　　70　　　　　　　75　　　　　　　80

His Asp Phe Phe Lys Ser Ala Met Pro Glu Gly Tyr Val Gln Glu Arg

　　　　　　85　　　　　　　90　　　　　　　95

Thr Ile Ser Phe Lys Asp Asp Gly Thr Tyr Lys Thr Arg Ala Glu Val

　　　　　　100　　　　　　105　　　　　　110

Lys Phe Glu Gly Asp Thr Leu Val Asn Arg Ile Glu Leu Lys Gly Ile

115     120     125

Asp Phe Lys Glu Asp Gly Asn Ile Leu Gly His Lys Leu Glu Tyr Asn

130     135     140

Phe Asn Ser His Asn Val Tyr Ile Thr Ala Asp Lys Gln Lys Asn Gly

145     150     155     160

Ile Lys Ala Asn Phe Lys Ile Arg His Asn Val Glu Asp Gly Ser Val

165     170     175

Gln Leu Ala Asp His Tyr Gln Gln Asn Thr Pro Ile Gly Asp Gly Pro

180     185     190

Val Leu Leu Pro Asp Asn His Tyr Leu Ser Thr Gln Ser Lys Leu Ser

195     200     205

Lys Asp Pro Asn Glu Lys Arg Asp His Met Val Leu Leu Glu Phe Val

210     215     220

Thr Ala Ala Gly Ile Thr Leu Gly Met Asp Glu Leu Tyr Lys

225     230     235

<210> 3

<211> 11

<212> PRT

<213> artificial sequence


<400> 3


Gly Ile Thr Leu Gly Met Asp Glu Leu Tyr Lys

1               5                    10


<210> 4

<211> 210

<212> PRT

<213> artificial sequence


<400> 4


Pro Ser Val Phe Leu Phe Pro Pro Lys Pro Lys Asp Thr Leu Met Ile

1               5                    10                    15


Ser Arg Thr Pro Glu Val Thr Cys Val Val Val Asp Val Ser Gln Glu

                20                   25                    30


Asp Pro Glu Val Gln Phe Asn Trp Tyr Val Asp Gly Val Glu Val His

                35                   40                    45

Asn Ala Lys Thr Lys Pro Arg Glu Glu Gln Phe Asn Ser Thr Tyr Arg

50 55 60

Val Val Ser Val Leu Thr Val Leu His Gln Asp Trp Leu Asn Gly Lys

65 70 75 80

Glu Tyr Lys Cys Lys Val Ser Asn Lys Gly Leu Pro Ser Ser Ile Glu

85 90 95

Lys Thr Ile Ser Lys Ala Lys Gly Gln Pro Arg Glu Pro Gln Val Tyr

100 105 110

Thr Leu Pro Pro Ser Gln Glu Glu Met Thr Lys Asn Gln Val Ser Leu

115 120 125

Thr Cys Leu Val Lys Gly Phe Tyr Pro Ser Asp Ile Ala Val Glu Trp

130 135 140

Glu Ser Asn Gly Gln Pro Glu Asn Asn Tyr Lys Thr Thr Pro Pro Val

145 150 155 160

Leu Asp Ser Asp Gly Ser Phe Phe Leu Tyr Ser Arg Leu Thr Val Asp

165 170 175

Lys Ser Arg Trp Gln Glu Gly Asn Val Phe Ser Cys Ser Val Met His

180 185 190

Glu Ala Leu His Asn His Tyr Thr Gln Lys Ser Leu Ser Leu Ser Leu

195 200 205

Gly Lys

210

<210> 5

<211> 22

<212> PRT

<213> artificial sequence

<400> 5

Ala Asp Asp Lys Glu Thr Cys Phe Ala Glu Glu Gly Lys Lys Leu Val

1 5 10 15

Ala Ala Ser Gln Ala Ala

20

<210> 6

<211> 22

<212> PRT

<213> artificial sequence


<400> 6


Ala Asp Asp Lys Glu Thr Gly Phe Ala Glu Glu Gly Lys Lys Leu Val

1               5                    10                   15


Ala Ala Ser Gln Ala Ala

          20


<210> 7

<211> 56

<212> PRT

<213> artificial sequence


<400> 7


Asp Lys Glu Trp Ile Leu Gln Lys Ile Tyr Glu Ile Met Arg Leu Leu

1               5                    10                   15


Asp Glu Leu Gly His Ala Glu Ala Ser Met Arg Val Ser Asp Leu Ile

          20                   25                   30

Tyr Glu Phe Met Lys Lys Gly Asp Glu Arg Leu Leu Glu Glu Ala Glu

       35            40            45

Arg Leu Leu Glu Glu Val Glu Arg

       50            55

<210> 8

<211> 160

<212> PRT

<213> artificial sequence

<400> 8

Ser Thr Ile Glu Glu Gln Ala Lys Thr Phe Leu Asp Lys Phe Asn His

1            5             10            15

Glu Ala Glu Asp Leu Phe Tyr Gln Ser Ser Leu Ala Ser Trp Asn Tyr

       20            25            30

Asn Thr Asn Ile Thr Glu Glu Asn Val Gln Asn Met Asn Asn Ala Gly

       35            40            45

Asp Lys Trp Ser Ala Phe Leu Lys Glu Gln Ser Thr Leu Ala Gln Met

       50            55           60

Tyr Pro Leu Gln Glu Ile Gln Asn Gly Gly Gly Gly Ser Gly Gly Gly

65    70    75    80

Gly Ser Thr Ser Gly Gly Val Thr Gly Thr Gln Gly Phe Trp Glu Asn

    85    90    95

Ser Met Leu Thr Asp Pro Gly Asn Val Gln Lys Ala Val Cys His Pro

   100    105    110

Thr Ala Trp Asp Leu Gly Lys Gly Asp Phe Arg Ile Leu Met Cys Thr

   115    120    125

Lys Val Thr Met Asp Asp Phe Leu Thr Ala His His Glu Met Gly His

   130    135    140

Ile Gln Tyr Asp Met Ala Tyr Ala Ala Gln Pro Phe Leu Leu Arg Asn

145    150    155    160

<210> 9

<211> 210

<212> PRT

<213> artificial sequence

<400> 9

Pro Ser Val Phe Leu Phe Pro Pro Lys Pro Lys Asp Thr Leu Met Ile
1               5                   10                  15

Ser Arg Thr Pro Glu Val Thr Cys Val Val Val Asp Val Ser Gln Glu
                20                  25                  30

Asp Pro Glu Val Gln Phe Asn Trp Tyr Val Asp Gly Val Glu Val His
        35                  40                  45

Asn Ala Lys Thr Lys Pro Arg Glu Glu Gln Phe Asn Ser Thr Tyr Arg
        50                  55                  60

Val Val Ser Val Leu Thr Val Leu His Gln Asp Trp Leu Asn Gly Lys
65                  70                  75                  80

Glu Tyr Lys Cys Lys Val Ser Asn Lys Gly Leu Pro Ser Ser Ile Glu
                85                  90                  95

Lys Thr Ile Ser Lys Ala Lys Gly Gln Pro Arg Glu Pro Gln Val Tyr
                100                 105                 110

Thr Leu Pro Pro Ser Gln Glu Glu Met Thr Lys Asn Gln Val Ser Leu

115                 120                 125

Thr Cys Leu Val Lys Gly Phe Tyr Pro Ser Asp Ile Ala Val Glu Trp

130                 135                 140

Glu Ser Asn Gly Gln Pro Glu Asn Asn Tyr Lys Thr Thr Pro Pro Val

145                 150                 155                 160

Leu Asp Ser Asp Gly Ser Phe Phe Leu Tyr Ser Arg Leu Thr Val Asp

165                 170                 175

Lys Ser Arg Trp Gln Glu Gly Asn Val Phe Ser Cys Ser Val Met His

180                 185                 190

Glu Ala Leu His Asn His Tyr Thr Gln Lys Ser Leu Ser Leu Ser Leu

195                 200                 205

Gly Lys

210

<210> 10

<211> 17

<212> PRT

<213> artificial sequence

<400> 10

Ile Thr Glu Thr Ser Ser Pro Phe Arg Ser Ile Phe Ser His Ser Gly

1 5 10 15

Lys

<210> 11

<211> 10

<212> PRT

<213> artificial sequence

<400> 11

Asp Asp Asp Asp Asp Asp Asp Asp Asp Asp

1 5 10

<210> 12

<211> 10

<212> PRT

<213> artificial sequence

<400> 12

Glu Glu Glu Glu Glu Glu Glu Glu Glu Glu

1            5            10

<210> 13

<211> 10

<212> PRT

<213> artificial sequence

<400> 13

Asp Glu Asp Glu Asp Glu Asp Glu Asp Glu

1            5            10

<210> 14

<211> 16

<212> PRT

<213> artificial sequence

<400> 14

Asp Asp Asp Asp Asp Asp Asp Asp Glu Glu Glu Glu Glu Glu Glu Glu

1            5            10            15

<210> 15

<211> 8

<212> PRT

<213> artificial sequence

<400> 15

His His His His His His His His

1 5

<210> 16

<211> 170

<212> PRT

<213> artificial sequence

<400> 16

Ser Thr Ile Glu Glu Gln Ala Lys Thr Phe Leu Asp Lys Phe Asn His

1 5 10 15

Glu Ala Glu Asp Leu Phe Tyr Gln Ser Ser Leu Ala Ser Trp Asn Tyr

20 25 30

Asn Thr Asn Ile Thr Glu Glu Asn Val Gln Asn Met Asn Asn Ala Gly

35 40 45

Asp Lys Trp Ser Ala Phe Leu Lys Glu Gln Ser Thr Leu Ala Gln Met

50 55 60

Tyr Pro Leu Gln Glu Ile Gln Asn Leu Thr Val Lys Leu Gly Gly Gly

65     70     75     80

Gly Ser Gly Gly Gly Gly Ser Thr Ser Gly Gly Val Thr Gly Gly Leu

    85     90     95

Pro Asn Met Thr Gln Gly Phe Trp Glu Asn Ser Met Leu Thr Asp Pro

    100     105     110

Gly Asn Val Gln Lys Ala Val Cys His Pro Thr Ala Trp Asp Leu Gly

    115     120     125

Lys Gly Asp Phe Arg Ile Leu Met Cys Thr Lys Val Thr Met Asp Asp

    130     135     140

Phe Leu Thr Ala His His Glu Met Gly His Ile Gln Tyr Asp Met Ala

145     150     155     160

Tyr Ala Ala Gln Pro Phe Leu Leu Arg Asn

    165     170

<210> 17

<211> 180

<212> PRT

<213> artificial sequence

<400> 17

Ser Thr Ile Glu Glu Gln Ala Lys Thr Phe Leu Asp Lys Phe Asn His

1               5                   10                  15

Glu Ala Glu Asp Leu Phe Tyr Gln Ser Ser Leu Ala Ser Trp Asn Tyr

            20                  25                  30

Asn Thr Asn Ile Thr Glu Glu Asn Val Gln Asn Met Asn Asn Ala Gly

        35                  40                  45

Asp Lys Trp Ser Ala Phe Leu Lys Glu Gln Ser Thr Leu Ala Gln Met

    50                  55                  60

Tyr Pro Leu Gln Glu Ile Gln Asn Leu Thr Val Lys Leu Gln Leu Gln

65                  70                  75                  80

Ala Leu Gly Gly Gly Gly Ser Gly Gly Gly Gly Ser Thr Ser Gly Gly

            85                  90                  95

Val Thr Gly Phe Phe Val Ser Val Gly Leu Pro Asn Met Thr Gln Gly

100                  105                  110

Phe Trp Glu Asn Ser Met Leu Thr Asp Pro Gly Asn Val Gln Lys Ala

115                  120                  125

Val Cys His Pro Thr Ala Trp Asp Leu Gly Lys Gly Asp Phe Arg Ile

130                  135                  140

Leu Met Cys Thr Lys Val Thr Met Asp Asp Phe Leu Thr Ala His His

145                  150                  155                  160

Glu Met Gly His Ile Gln Tyr Asp Met Ala Tyr Ala Ala Gln Pro Phe

165                  170                  175

Leu Leu Arg Asn

180

<210> 18

<211> 190

<212> PRT

<213> artificial sequence

<400>  18

Ser Thr Ile Glu Glu Gln Ala Lys Thr Phe Leu Asp Lys Phe Asn His

1                5                10                15

Glu Ala Glu Asp Leu Phe Tyr Gln Ser Ser Leu Ala Ser Trp Asn Tyr

20                25                30

Asn Thr Asn Ile Thr Glu Glu Asn Val Gln Asn Met Asn Asn Ala Gly

35                40                45

Asp Lys Trp Ser Ala Phe Leu Lys Glu Gln Ser Thr Leu Ala Gln Met

50                55                60

Tyr Pro Leu Gln Glu Ile Gln Asn Leu Thr Val Lys Leu Gln Leu Gln

65                70                75                80

Ala Leu Gln Gln Gly Gly Gly Gly Ser Gly Gly Gly Gly Ser Thr Ser

85                90                95

Gly Gly Val Thr Gly Arg Ile Phe Lys Glu Ala Glu Lys Phe Phe Val

100                105                110

Ser Val Gly Leu Pro Asn Met Thr Gln Gly Phe Trp Glu Asn Ser Met

115               120               125

Leu Thr Asp Pro Gly Asn Val Gln Lys Ala Val Cys His Pro Thr Ala

130            135           140

Trp Asp Leu Gly Lys Gly Asp Phe Arg Ile Leu Met Cys Thr Lys Val

145          150           155          160

Thr Met Asp Asp Phe Leu Thr Ala His His Glu Met Gly His Ile Gln

165          170           175

Tyr Asp Met Ala Tyr Ala Ala Gln Pro Phe Leu Leu Arg Asn

180          185          190

<210> 19

<211> 200

<212> PRT

<213> artificial sequence

<400> 19

Ser Thr Ile Glu Glu Gln Ala Lys Thr Phe Leu Asp Lys Phe Asn His

1          5          10          15

Glu Ala Glu Asp Leu Phe Tyr Gln Ser Ser Leu Ala Ser Trp Asn Tyr

          20                    25               30

Asn Thr Asn Ile Thr Glu Glu Asn Val Gln Asn Met Asn Asn Ala Gly

      35                 40               45

Asp Lys Trp Ser Ala Phe Leu Lys Glu Gln Ser Thr Leu Ala Gln Met

     50               55             60

Tyr Pro Leu Gln Glu Ile Gln Asn Leu Thr Val Lys Leu Gln Leu Gln

65              70              75             80

Ala Leu Gln Gln Gly Gly Gly Gly Ser Gly Gly Gly Gly Ser Thr Ser

        85              90             95

Gly Gly Val Thr Gly Gly His Ala Gly Gly Thr Val Asp Ala Gln Arg

      100             105            110

Ile Phe Lys Glu Ala Glu Lys Phe Phe Val Ser Val Gly Leu Pro Asn

      115             120            125

Met Thr Gln Gly Phe Trp Glu Asn Ser Met Leu Thr Asp Pro Gly Asn

130                135                140

Val Gln Lys Ala Val Cys His Pro Thr Ala Trp Asp Leu Gly Lys Gly

145             150            155             160

Asp Phe Arg Ile Leu Met Cys Thr Lys Val Thr Met Asp Asp Phe Leu

165           170           175

Thr Ala His His Glu Met Gly His Ile Gln Tyr Asp Met Ala Tyr Ala

180           185           190

Ala Gln Pro Phe Leu Leu Arg Asn

195           200

**Description**

**TECHNICAL FIELD**

The present disclosure pertains to the biotechnology field, and specifically relates to a polymerizing molecule, a monomer structure and a polymer structure comprising the same, and related products, preparation methods, and uses thereof.

**BACKGROUND**

The virus cell receptor is the gateway for the virus to invade the target cells, and can bind to the virus specifically. The virus cell receptor mediates the invading of virus into the susceptible host cells and to initiate the replication process of the virus, thus endangering the host.

For example, the COVID-19 virus will infect Human cells via the human ACE2. For the COVID-19 virus, the human ACE2 receptor serves as a "doorknob", which binds to the spike protein of the virus, thus opening the door to infect human cells.

In a study entitled "Structural insights into hepatitis C virus receptor binding and entry" published in Nature, researchers from the National Institute of Allergy and Infectious Diseases (NIAID) of the National Institute of Health of the United States have structurally revealed the mechanism by which hepatitis C virus enters cells and clearly described the process during which HCV-glycoprotein E2 interacts with CD81, i.e. the process during which the HCV enters and infects human cells. Scientists have found that under acidic conditions, HCV E2 readily binds to the CD81 receptor, and once the virus interacts with the receptor, HCV E2 changes its configuration (after binding to CD81, the residues 418 to 422 in HCV E2 is translocated, which extends the inner ring consisting of the residues 520 to 539), making the virus in closer contact with the cell membrane, thereby facilitating its entry into the cells.

Hepatitis B virus (HBV) and its satellite virus, Hepatitis D virus (HDV), must bind to cell surface receptor molecules to infect host cells.

Rabies virus (RV), a member of the genus Lyssavirus, family Rhabdoviridae, is highly neurotropic and can cause fatal encephalitis. Currently, there is no effective treatment method, and fatality rate is almost 100%. Rabies virus encodes five proteins in which G protein plays an important role in some areas, such as determining the host range, neurovirulence, and immunogenicity of the virus, and interaction with host cell surface receptor molecules. The nAchR located in the postsynaptic membrane and the NCAM located in the presynaptic membrane play important mediating roles when RV invades the neuromuscular junction. The P75 neurotrophin receptor is also a receptor for rabies virus. When the virus enters the neuronal cells, P75 can bind to the rabies virus G protein and causes the virus to enter the cytoplasm for reverse transmission.

Currently, these viruses are generally prevented and treated by using species such as a macromolecule antibody or a nanobody.

For example, there are several antibody-based drugs for treating COVID-19 pneumonia: 1. neutralizing antibodies against S protein, which bind to the S protein on the surface of the virus particles, blocking the binding of S protein to ACE2, and thereby blocking virus entry into the cells; 2. neutralizing antibodies against the ACE2 protein, which bind to the receptor ACE2 of the virus, blocking virus entry into the cells; 3. ACE2 analogue, which competes with the ACE2 on the surface of the lung cells to bind the S protein on the surface of the virus particles, blocking the binding of the virus to the receptor, and; 4. Antibodies against cytokine storm, which inhibit cytokine storm, thus treating COVID-19 pneumonia.

However, the antibody-based drugs are highly costly in production than the small molecule drugs and need to be generated by using a biological pharmaceutical method with more time and more materials. Therefore, the antibody-based medicines cannot be comparable to the small molecule drugs.

In addition, currently, the antibodies are usually monovalent or divalent antibodies which have relatively insufficient capability for capturing the virus.

**SUMMARY**

The present disclosure provides a polymerizing molecule, a monomer structure and a polymer structure including the same, and related products, preparation methods, and uses thereof. A polymer that can block binding to the receptor is formed by the polymerization of the polymerizing molecules so as to effectively improve the capacity of binding to the virus.

Therefore, the present disclosure provides the following technical solutions.

The present disclosure provides a polymerizing molecule, characterized in that, the polymerizing molecule is configured to polymerizing a plurality of binding-blocking molecular units that block binding between a virus and a cell receptor into a polymer structure.

The polymerizing molecule provided in the present disclosure is further characterized in that the polymerizing molecule has a monomer binding site and a polymerizing site monomer binding site. The monomer binding site is configured to bind to a binding-blocking molecular unit so as to form a monomer structure having at least the polymerizing molecule and the binding-blocking molecular unit. A polymer structure is formed by polymerizing a plurality of the monomer structures through the polymerizing site. Preferably, when the polymerizing molecule is a peptide or a protein, the monomer binding site is located at the N-terminus of the polymerizing molecule.

The polymerizing molecule provided in the present disclosure is further characterized in that the polymer structure is formed by polymerizing 2 to 10 monomer structures.

The polymerizing molecule provided in the present disclosure is further characterized in that the polymerizing molecule is any one selected from Table 1.

The polymerizing molecules provided in the present disclosure is further characterized in that the polymerizing molecule is streptavidin. Preferably, streptavidin comprises an amino acid sequence that is identical to SEQ ID NO: 1 or has at least 50%, 60%, 70, 80%, 85%, 90%, 95% or 99% identity to SEQ ID NO: 1.

The polymerizing molecule provided in the present disclosure is further characterized in that the polymerizing molecule binds to the binding-blocking molecule unit via a linker molecule.

The linker molecule comprises any one or more of a fluorescent protein, a human immunoglobulin G4, a Fc, and an HSA. For example, the linker molecule is fluorescent protein eGFP or is obtained by means of modification of the fluorescent protein eGFP, preferably by deleting some amino acids from eGFP.

Alternatively, the linker molecule comprises an amino acid sequence that is identical to any one of SEQ ID NOs: 2 to 6 or has at least 50%, 60%, 70, 80%, 85%, 90%, 95% or 99% identity to any one of SEQ ID NOs: 2 to 6, preferably obtained by deleting the amino acids at positions 1 to 228 from eGFP.

Preferably, the linker molecule has an N-terminus that is linked to the binding-blocking molecule unit, and a C-terminus that is linked to the N-terminus of the polymerizing molecule.

The polymerizing molecule provided in the present disclosure is further characterized in that the binding-blocking molecule unit blocks the binding between the virus and a cell receptor by binding to a site of the virus at which the virus binds to the cell receptor, and/or the binding-blocking molecule unit blocks the binding between the virus and the cell receptor by binding to the cell receptor.

The polymerizing molecule provided in the present disclosure is further characterized in that the binding-blocking molecule unit comprises at least one blocking molecule that blocks binding between a virus and a cell receptor. Preferably, the blocking molecule comprises an amino acid sequence that is identical to any one of SEQ ID NOs: 7 to 9 and 16 to 19 or has at least 50%, 60%, 70, 80%, 85%, 90%, 95% or 99% identity to any one of SEQ ID NOs: 7 to 9 and 16 to 19. Further, when the binding-blocking molecule unit comprises a plurality of blocking molecules, the blocking molecules are linked sequentially to each other in a direction from the N-terminus to the C-terminus. More preferably, when the binding-blocking molecule unit is linked to the polymerizing molecule via the linker molecule, the N-terminus of the linker molecule is linked to the C-terminus of the last blocking molecule, and the C-terminus of the linker molecule is linked to the monomer binding site. Still more preferably, the blocking molecule is a nanobody.

The polymerizing molecule provided in the present disclosure is further characterized in that the cell receptor is ACE2. Preferably, the blocking peptide is an ACE2 analog.

The polymerizing molecule provided in the present disclosure is further characterized in that the binding-blocking molecule unit comprises at least one first blocking molecule and/or at least one second blocking molecule. The first blocking molecule comprises an amino acid sequence that is identical to SEQ ID NO: 7 or has at least 50%, 60%, 70, 80%, 85%, 90%, 95% or 99% identity to SEQ ID NO: 7, and the second blocking molecule comprises an amino acid sequence that is identical to any one of SEQ ID NOs: 8 and 16 to 19 or has at least 50%, 60%, 70, 80%, 85%, 90%, 95% or 99% identity to any one of SEQ ID NOs: 8 and 16 to 19. Preferably the binding-blocking molecule unit comprises two first blocking molecules or two second blocking molecules. More preferably, the two first blocking molecules or the two second blocking molecules are linked to each other respectively via a N-terminus of one to a C-terminus of the other. Still more preferably, the binding-blocking molecule unit comprises the first blocking molecule and the second blocking molecule of which the N-terminus of one is linked to the C-terminus of the other, more preferably, the N-terminus of the second blocking molecule is linked to the C-terminus of the first blocking molecule.

The polymerizing molecule provided in the present disclosure is further characterized in that the monomer structure further comprises a leading peptide that is configured to promote the production of the polymer structure by expression in the cells or secretion from the cells, or by in vitro cell-free expression. The leading peptide comprises an amino acid sequence that is identical to SEQ ID NO: 10 or has at least 50%, 60%, 70, 80%, 85%, 90%, 95% or 99% identity to SEQ ID NO: 10. Preferably, the leading peptide has a C-terminus that is linked to the N-terminus of any one of the blocking molecules.

The polymerizing molecule provided in the present disclosure is further characterized in that the monomer structure further comprises an acidic structure. The acidic structure is a short-chain polymer of amino acids which is negatively charged. Further, the acidic structure has one or more of the following characteristics:

(1) the acidic structure is located at the C-terminus;

(2) the short-chain polymer has 0 to 50, 2 to 40, 3 to 30, 2 to 20, or 2 to 10 amino acid residues; and

(3) the negatively charged amino acids are aspartate and/or glutamate,

Preferably, the acidic structure is linked to the C-terminus of the polymerizing molecule.

The polymerizing molecule provided in the present disclosure is further characterized in that the monomer structure further comprises a protein tag. The protein tag comprises an amino acid sequence that is identical to SEQ ID NO: 15 or has at least 50%, 60%, 70, 80%, 85%, 90%, 95% or 99% identity to SEQ ID NO: 15. Preferably, the protein tag has a C-terminal that is linked to the N-terminal of a binding-blocking molecule unit. When the binding-blocking molecule unit is blocking peptide fragments, the C-terminal of the protein tag is linked to the N-terminal of any one of blocking peptide fragments. More preferably, when the monomer structure further comprises the leading peptide, the leading peptide is linked to the binding-blocking molecule unit via the protein tag.

The polymerizing molecule provided in the present disclosure is further characterized in that the virus is one or more of hepatitis B virus, rabies virus, HVP, and COVID-19 virus.

The present disclosure further provides a monomer structure, characterized in that the monomer structure comprises the above-mentioned polymerizing molecule. Preferably, the monomer structure has a size of 30 to 80 KD, and is the above-mentioned monomer structure.

The present disclosure further provides a polymer structure, characterized in that the polymer structure is formed by polymerizing a plurality of the above-mentioned monomer structures, and the polymer structure is the above-mentioned polymer structure.

The polymer structure provided in the present disclosure is further characterized in that the polymer structure is formed by polymerizing at least four monomer structures. Preferably, the polymer structure is formed by polymerizing four monomer structures.

The polymer structure provided in the present disclosure is further characterized in that the blocking structure unit is a blocking polypeptide and has a binding force that is 1000 to 1,000,000 times greater than a nanobody.

The polymer structure provided in the present disclosure is further characterized in that the polymer structure is soluble.

The polymer structure provided in the present disclosure is further characterized in that the polymer structure is thermally stable at a temperature that is greater than or equal to 45°C.

The present disclosure further provides a nucleic acid that encodes the polymerizing molecule, the monomer structure, or the polymer structure, characterized in that, the polymerizing molecule is the above-mentioned polymerizing molecule, the monomer structure is the above-mentioned monomer structure; and the polymer structure is the above-mentioned polymer structure.

The present disclosure further provides a vector that comprises the above-mentioned nucleic acid.

The present disclosure further provides a eukaryotic host cell for expression in cells, secretion from cells or in vitro cell-free synthesis and expression of the monomer structure or the polymer structure, wherein the monomer structure is the above-mentioned monomer structure, and the polymer structure is the above-mentioned polymer structure.

The present disclosure further provides a use of the above-mentioned nucleic acid or the above-mentioned vector in a method for preparing the monomer structure and/or the polymer structure.

The present disclosure further provides a use of any one of the polymerizing molecule, the monomer structure, and the polymer structure in: a medicament for treating a virus, detection and diagnosis of a virus, a medical use, a disinfection product against a virus, a cosmetic product, a skin care product, a care product, food, or a cleaning product, wherein the polymerizing molecule is the above-mentioned polymerizing molecule; and the monomer structure is the above-mentioned monomer structure;

The polymer structure is the above-mentioned polymer structure. Preferably, an unpurified product obtained by in vitro cell-free synthesis of the polymer structure is directly applied for the use.

The present disclosure further provides a disinfection product, a cosmetic product, a cosmetic product, a skin care product, a care product, a food or a cleaning product, characterized in that, comprising one or more of any one of the polymerizing molecule, the monomer structure, or the polymer structure, wherein the polymerizing molecule is the above-mentioned polymerizing molecule; the monomer structure is the above-mentioned monomer structure; and the polymer structure is the above-mentioned polymer structure.

The present disclosure further provides a medicament, characterized in that, comprising: one or more of the polymerizing molecules, the monomer structure, and the polymer structure; and a pharmaceutically acceptable carrier, diluent, or excipient, wherein the polymerizing molecule is the above-mentioned polymerizing molecule; the monomer structure is the above-mentioned monomer structure; and the polymer structure is the above-mentioned polymer structure.

The present disclosure further provides a method for preparing the polymer structure, and the polymer structure is obtained by in vitro cell-free synthesis and expression, characterized in that, when protein purification is performed with ammonium sulfate precipitation method on a product comprising a polymer structure obtained in a reaction, wherein the polymer structure is the above-mentioned polymer structure.

Functions and Effects

(1) The polymerizing molecule provided in the present disclosure allows the effective polymerization of a plurality of binding-blocking molecular units that block the binding between the virus and the cell receptor. In this manner of blocking the binding between the virus and the cell receptor, since the number of binding-blocking molecular units in each polymer structure is increased due to the polymerization of the polymerizing molecules, the binding between the virus and the cell receptor is blocked at multiple sites. Meanwhile, since a plurality of binding-blocking molecular units can bind to a plurality of viruses at the same time, and a plurality of polymer structures cooperate, which will lead to cross-linking, thus binding between the virus and the cell receptor will be effectively blocked. It can be learned from experiments that, compared with that before polymerization, the virus-blocking effect is improved after polymerization with a binding force that is 1000 to 1000,000 times greater than that of a nanobody. In this manner, the required number of binding molecules is less and the production cost is lower, compared with binding to a single site.

(2) Further, when at least four monomer structures that include the above-mentioned polymerizing molecule are mutually polymerized into a polymer structure by the respective polymerizing molecule, a structure of at least a tetrahedron is formed since each polymer structure has at least four monomer structures, so as to achieve crosslinking effect more effectively and to further improve the foregoing blocking effect. Preferably, compared with those formed by polymerizing more than four monomer structures, the polymer structure formed by polymerizing four monomer structures has an appropriate size, and ensures a sufficient blocking capability, without wasted spatial binding sites or space.

(3) Further, the streptavidin provided in the present disclosure enables the polymerization of monomer structures by the polymerizing molecule, and more than 90% of the polymer structures is those formed by polymerizing four monomer structures.

(5) Further, the binding between the virus and the cell receptor can be blocked by the binding-blocking molecule in the form of a peptide fragment. Compared with antibodies, the entire monomer structure or the polymer structure has a smaller size and is a simpler structure, which is more suitable for obtaining by means of expression in cells, secretion from cells, or in-vitro expression.

(6) Further, there are a plurality of blocking molecules, which can increase the possibility of blocking the binding between the virus and the cell receptor. Further, the first blocking molecule and/or the second blocking molecule have a better blocking effect on the binding between the virus and the cell receptor.

(7) Further, when the monomer structure further comprises a leading peptide that is configured to promote the production of the polymer structure by expression in the cells or secretion from the cells, or by in vitro cell-free expression, it is more advantageous to produce the above-mentioned monomer structure or the above-mentioned polymer structure in a manner of expression in the cells or secretion from the cells, or by in vitro cell-free expression.

(8) Further, when the binding-blocking molecule unit is linked to the polymerizing molecule by using a fluorescent protein or a linker molecule obtained by modifying the fluorescent protein, the virus-blocking effect can be improved. Further, the monomer structure further includes an acidic structure. The presence of the acid structure can improve the virus-blocking effect, and can improve thermal stability of the polymer structure.

**BRIEF DESCRIPTION OF THE DRAWINGS**

FIGs. 1 to 22 are diagrams of experimental results of inhibition of virus-blocking protein against pseudoviruses involved in example 2;

FIG. 23 is an electrograph of a virus-blocking protein before and after action on pseudoviruses in example 2;

FIG. 24 is a three-dimensional schematic diagram of a polymer structure according to example 1;

FIG. 25 is a three-dimensional schematic diagram of a cross-linking effect of a polymer structure with a virus according to example 1.

## DETAILED DESCRIPTION

Specific embodiments of the present disclosure are described with reference to the accompanying drawings. For specific methods or materials used in the embodiments, a person skilled in the art may make a conventional alternative selection based on the technical idea of the present disclosure and according to the existing technology, but is not limited to the specific description in the examples of the present disclosure.

The methods used in the examples are all conventional methods unless otherwise specified. The materials, reagents or the like used herein are commercially available unless otherwise specified.

The term "coronavirus" in the present disclosure refers to a coronavirus that is classified systematically to genus Coronavirus, family Coronaviridae, order Nidovirales and takes ACE2 as a binding receptor, including but not limited to SARS-CoV, MERS-CoV, SARS-CoV-2, and the like.

A spike protein, i.e., an S protein, is a type of marker transmembrane protein on a virus surface, and has two subunits: S1 on which a receptor binding site (RBD) is located, and S2. The spines on the outer membrane of virus particles is formed by the spike protein in a form of trimers, which has the main function of recognizing the surface receptor of a host cell and mediating fusion with the host cell.

The ACE2 (also known as ACAH) in the present disclosure is also referred to as angiotensin converting enzyme 2. ACE2 consists of 805 amino acids and is a type I transmembrane glycoprotein with a single extracellular catalytic domain. ACE2 is a receptor protein through which coronavirus, such as SARS-Cov-2, invades human cells.

In the present disclosure, in vitro cell-free expression, i.e. cell-free protein synthesis system, is a system based on transcription-translation coupling in prokaryotic or eukaryotic cells, referring to synthesis of polypeptides or other macromolecules in a reaction mixture comprising biological extracts and/or determined reagent.

The "D2P" system in the present disclosure includes but is not limited to an IVTT reaction (in vitro transcription and translation reaction). In the present disclosure, the IVTT reaction is preferred. The IVTT reaction, corresponding to the IVTT system, is a process of translating DNA into protein in vitro. Therefore, we also refer to the protein synthesis system in vitro as, D-to-P system, D_to_P system, and DNA to-Protein system. The corresponding method for in vitro protein synthesis is also referred to as D2P method, D-to-P method, D_to_P method, and DNA-to-Protein method, which has the same meaning as "in vitro cell-free protein synthesis system", "in vitro expression system", "in vitro protein synthesis system", "in vitro protein synthesis reaction system", "cell-free protein synthesis system", etc. Protein in vitro synthesis system, in vitro protein synthesis system, cell-free system, cell-free protein synthesis system, cell-free in vitro protein synthesis system, in vitro cell-free protein synthesis system, in vitro cell-free synthesis system, CFS system (cell-free system), or CFPS system (cell-free protein synthesis system) comprises in vitro translation system, in vitro transcription and translation system (IVTT system), etc. We also refer to the in vitro protein synthesis system as the "protein synthesis factory " (Protein Factory). In vitro protein synthesis reaction refers to the reaction during the protein synthesis in an in vitro cell-free synthesis system, including at least the translation process.

In the present disclosure, a protein component (for example, an RNA polymerase) required in an in vitro cell-free protein synthesis system may be provided in an endogenous manner, or may be added in an exogenous manner. When provided in an endogenous manner, reference may be made to genetic engineering, which includes, but is not limited to, inserting a coding sequence into an intracellular free plasmid, integrating a coding gene into a cell genome, and a combination thereof, provided in prior documents including, but not limited to, CN108690139A, CN109423496A, CN106978439A, CN110408635A, CN110551700A, CN110093284A, CN110845622A, CN110938649A, CN111378708A, CN111484998A, "Molecular and Cellular Biology, 1990, 10(1) :353-360", etc., and their reference. When provided in an exogenous manner, the amount can be controlled and adjusted as needed in the system.

The Host cell is well-known in the art, including but not limited to Escherichia coli, CHO cells, Chinese hamster ovary, NS0, SP2 cells, HeLa cells, baby hamster kidney (BHK) cells, monkey kidney (COS) cells, human liver cell carcinoma cells (e.g., Hep G2), A549 cells, HEK-293 cells, and many other cell lines. Spodoptera frugiperda or Trichoplusiani, amphibian cells, bacterial cells, plant cells, and fungal cells. The fungal cells include yeast and filamentous fungal cells, and the filamentous fungal cells include, for example, Pichia pastoris, Pichia finlandica, Pichia trehalophila, Pichia koclamae, Pichia membranaefaciens, Pichia minuta (Ogataeaminuta), Pichia lindneri, Pichia opuntiae, Pichia thermotolerans, Pichia salictaria, Pichia g uercuum, Pichia pijperi, Pichiastiptis, Pichia methanolica, Pichia sp., Saccharomyces cerevisiae, Saccharomyces sp., Hansenulapolymorpha, Kluyveromyces sp., Kluyveromyceslactis, Candida albicans, Aspergillus nidulans, Aspergillus niger, Aspergillus oryzae, Trichoderma reesei, Chrysosporium lucknowense, Fusarium sp., Fusarium gramineum, Fusarium venenatum, Physcomitrellapatens and Neurospora crassa.

Further, the in vitro cell-free protein synthesis system includes but is not limited to an in vitro protein synthesis system in Escherichia coli, an in vitro protein synthesis system in bacterium, an in vitro protein synthesis system in mammal cells (such as HF9, Hela, CHO, and HEK293 cells), an in vitro protein synthesis system in plant cells, an in vitro protein synthesis system in yeast cells, and an in vitro protein synthesis system in insect cells. Yeast cells are any one or more selected from the group consisting of Saccharomyces cerevisiae, Pichia pastoris, and Kluyveromyces. For example, Kluyveromyces is Kluyveromyceslactis.

The technical elements of the present disclosure such as the in vitro protein synthesis system, the template, the plasmid, the target protein, the in vitro protein synthesis reaction (incubation reaction), various preparation methods, and various detection methods may also be independently selected from the following documents: CN111484998A, CN106978349A, CN108535489A, CN108690139A, CN108949801A, CN108642076A, CN109022478A, CN109423496A, CN109423497A, CN109423509A, CN109837293A, CN109971783A, CN109988801A, CN109971775A, CN110093284A, CN110408635A, CN110408636A, CN110551745A, CN110551700A, CN110551785A, CN110819647A, CN110845622A, CN110938649A, and CN110964736A. Unless conflicting with the object of the present disclosure, these documents and their references are incorporated herein by reference in their entirety for all purposes.

In the present disclosure, when reference to blocking the binding between a virus and a cell receptor, it refers to inactivating the virus, for example, in the following manner.

A manner of reducing the binding between the virus and the cell receptor includes:

The first manner belongs to competitively blocking, that is, binding a receptor analogue to the virus so as to reduce the possibility of the virus binding to the receptor cell; alternatively, reducing the cell receptor that can bind to the virus with a molecule that binds to the receptor so as to reduce the possibility of the virus binding to the receptor cell;

The second manner is blocking by crosslinking formed between different viruses by using the binding-blocking molecule unit as a medium to reduce the possibility of the virus binding to the receptor cell;

Another manner is destroying the functional region that bind to the cell receptor by using an antibody so as to achieve blocking.

"The polymerizing molecule" herein refers to molecule(s) that can be polymerized together by, for example, intermolecular forces, or by self-association between specific domains.

The "monomer structure" herein refers to a monomer structure of the polymerizing molecule before polymerization, that is, a structure having at least the binding-blocking molecular unit and the polymerizing molecule, also referred to as a monomer.

The "polymer structure" herein refers to a structure formed by polymerizing a plurality of monomer structures together by means of binding between polymerizing molecules, which is also referred to as a polymer.

Example 1

In this example, the present disclosure is specifically described.

This example provides a polymerizing molecule, characterized in that, the polymerizing molecule is configured to polymerizing the binding-blocking molecular units that block binding between a virus and a cell receptor into a polymer structure. The binding-blocking molecular units herein comprise a binding molecule that bind to the cell receptor or the virus, thereby enabling competitively blocking the binding of the viral to the cell receptor.

To facilitate elaboration, the polymerizing molecule herein is denoted by A, and the binding-blocking molecule unit herein is denoted by B.

Since a plurality of binding-blocking molecule units are polymerized due to the polymerization of the polymerizing molecules, the number of binding-blocking molecular units in the polymer structure is increased, that is, the sites at which the competitively blocking occurs are increased and the binding between the virus and the cell receptor is blocked at multiple sites. Meanwhile, since a plurality of binding-blocking molecular units can bind to a plurality of viruses at the same time, leading to cross-linking, binding between the viruses and the cell receptors will be effectively blocked.

Specifically, the polymerizing molecule in this example has a monomer binding site and a polymerizing site.

The monomer binding site is configured to bind to a binding-blocking molecular unit so as to form a monomer structure. A polymer structure is formed by polymerizing a plurality of the monomer structures through the polymerizing site. That is, the polymerizing site in different monomer structures has a function of binding the monomer structures together, for example, by a self-association binding under an intermolecular force. Preferably, when the polymerizing molecule is a peptide or a protein, the monomer binding site is located at the N-terminus of the polymerizing molecule. That is, the C-terminus of the binding-blocking molecule unit is linked to the N-terminus of the polymerizing molecule. Specifically, the monomer structure has a basic structure as shown in Formula 1:

B-A (Formula 1): Preferably, the monomer binding site is located at a C-terminus of the polymerizing molecule.

Preferably, the polymer structure is formed by the polymerization of at least four monomer structures. When at least four monomer structures that include the above-mentioned polymerizing molecule are mutually polymerized into a polymer structure by the respective polymerizing molecule, a structure of at least tetrahedron is formed since each polymer structure has at least four monomer structures, so as to achieve crosslinking effect more effectively and to further improve the foregoing blocking effect. Preferably, compared with those formed by polymerizing more than four monomer structures, the polymer structure formed by polymerizing four monomer structures has an appropriate size, and ensures a sufficient blocking capability, without wasted spatial binding sites or space.

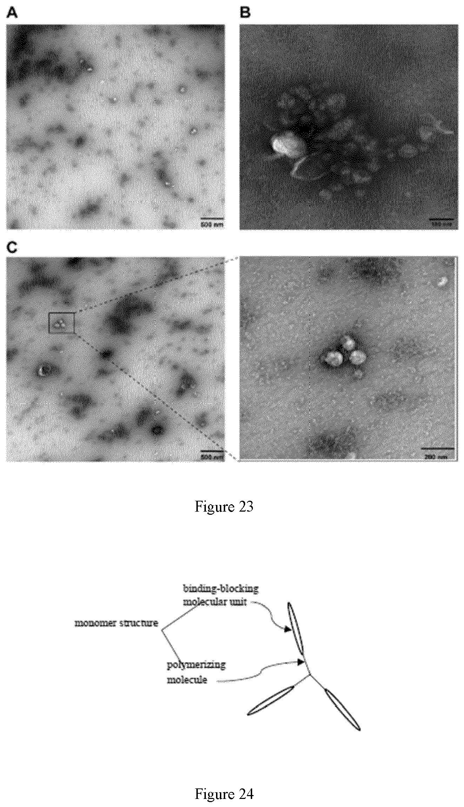

FIG. 24 is a three-dimensional schematic diagram of a polymer structure according to example 1.

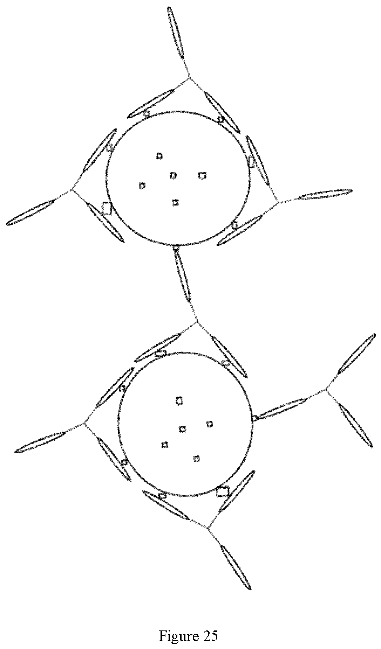

FIG. 25 is a three-dimensional schematic diagram of a cross-linking effect of a polymer structure with a virus according to example 1.

As shown in FIG. 24, the polymer structure in this figure is formed into a tetrahedron structure by polymerizing three monomer structures, with an intermediate part polymerized through the polymerizing sites and each binding-blocking molecular unit distributed at three points.

As shown in FIG. 25, the polymer structures in this figure are formed by polymerizing a single monomer structure. In each of the polymer structures, two binding-blocking molecules that bind to a same virus and the other binding-blocking molecule that bind to another virus. Therefore, cross-linking is formed between the two viruses through the binding-blocking molecule, which can block other viruses in contact with a cell receptor constantly, thereby improving the blocking effect.

In this example, the polymerizing molecule is selected from any one of Table 1:

| Table 1 | | |
|---|---|---|
| No. | protein name | gene name |
| 1 | cellular tumor antigen P53 (antigen NY-CO-13) | TP53 P53 |
| 2 | BTB/POZ domain-containing protein KCTD1 (Potassium channel polymerizing site -containing protein 1) | KCTD1 C18orf5 |
| 3 | potassium voltage-gated channel subfamily KQT member 1 | KCNQ1 KCNA8 KCNA9 KVLQT 1 |
| 4 | Protein CBFA2T1 (Cyclin-D-related protein) (821 Protein) (Protein ETO) (Protein MTG8) (Zinc finger MYND domain-containing protein) | RUNX1T1 AML1T1CBFA2T1CDRETOMTG8 ZMYND2 |
| 5 | BTB/POZ domain-containing adapter for CUL3-mediated RhoA degradation protein 3 (hBACURD3) (BTB/POZ domain-containing protein 10) (Potassium channel tetramerization domain -containing protein 10) | KCTD 10 ULR061 MSTP028 |
| 6 | potassium voltage-gated channel subfamily H member 2 (Eag homologue) (ether-a-go-go-related gene potassium channel 1) (ERG-1) (Eag related protein 1) (ether -a-go-go-related protein 1) (H-ERG) | KCNH2 EGR EGR1 HERG |
| 7 | C-C motif chemokine 5 (EoCP) | CCL5 D17S136ESA5 |
| 8 | Streptavidin | |

In an illustrative example, the polymerizing molecule is streptavidin or comprises an amino acid sequence that is identical to SEQ ID NO: 1 or has at least 50%, 60%, 70, 80%, 85%, 90%, 95% or 99% identity to SEQ ID NO: 1. With such a polymerizing molecule, tetrameric structures (formed by polymerizing four monomer structure) generated by the polymerization is 80% or more, or even 90% or more.

In an illustrative example, the polymerizing molecule can bind to the binding-blocking molecule unit otherwise via a linker molecule. To facilitate elaboration, the linker molecule is denoted by D.

In an illustrative example, the linker molecule comprises any one or more of a fluorescent protein, a human immunoglobulin G4, a Fc, and an HSA. For example, the linker molecule is a fluorescent protein eGFP or obtained by modifying the fluorescent protein eGFP, preferably by deleting some amino acid residues of eGFP. For example, the linker molecule is a fluorescent protein eGFP or obtained by modifying the fluorescent protein eGFP, preferably by deleting amino acid residues at positions 1 to 228 of the eGFP.

Preferably, the linker molecule comprises an amino acid sequence that is identical to any one of SEQ ID NO: 2 or has at least 50%, 60%, 70, 80%, 85%, 90%, 95% or 99% identity to any one of SEQ ID NO: 2, or the linker molecule comprises an amino acid sequence that is identical to any one of SEQ ID NO: 3 or has at least 50%, 60%, 70, 80%, 85%, 90%, 95% or 99% identity to any one of SEQ ID NO: 3, or the linker molecule comprises an amino acid sequence that is identical to any one of SEQ ID NO: 4 or has at least 50%, 60%, 70, 80%, 85%, 90%, 95% or 99% identity to any one of SEQ ID NO: 4, or the linker molecule comprises an amino acid sequence that is identical to any one of SEQ ID NO: 5 or has at least 50%, 60%, 70, 80%, 85%, 90%, 95% or 99% identity to any one of SEQ ID NO: 5, or the linker molecule comprises an amino acid sequence that is identical to any one of SEQ ID NO: 6 or has at least 50%, 60%, 70, 80%, 85%, 90%, 95% or 99% identity to any one of SEQ ID NO: 6.

In an illustrative example, the linker molecule has an N-terminus that is linked to the binding-blocking molecule unit, and a C-terminus that is linked to a monomer binding site of the polymerizing molecule at its N-terminus. In this case, the monomer structure has a structure as shown in Formula 2:

B-D-A (Formula 2). Preferably, the C-terminus of B is linked to the N-terminus of D, and the N-terminus of D is linked to the C-terminus of A.

In an illustrative example, the binding-blocking molecule unit comprises at least one blocking molecule that binds to a receptor binding region of a virus. That is, the binding-blocking molecule unit may comprise one blocking molecule, or a plurality of blocking molecules. When the binding-blocking molecule unit comprises a plurality of blocking molecules, these blocking molecules may have the same or different amino acid sequences.

A plurality of blocking molecules may increase the possibility of blocking the binding between the virus and the cell receptor, thereby improving the above-mentioned blocking effect.

In an illustrative example, when there is a plurality of blocking molecules, the blocking molecules are linked sequentially to each other in a direction from the C-terminus to the N-terminus. That is, the C-terminus of the former is linked to the N-terminus of the latter, specifically B1-B2-B3...Bn (n is a positive integer greater than 1). More preferably, when the binding-blocking molecule unit is linked to the polymerizing molecule via the linker molecule, the N-terminus of the linker molecule is linked to the C-terminus of the last blocking molecule, and the C-terminus of the linker molecule D is linked to the polymerizing molecule, specifically B1-B2-B3...Bn-D-A.

In an illustrative example, the blocking molecule comprises an amino acid sequence that is identical to any one of SEQ ID NOs: 7 to 9 and 16 to 19 or has at least 50%, 60%, 70, 80%, 85%, 90%, 95% or 99% identity to any one of SEQ ID NOs: 7 to 9 and 16 to 19. The detailed explanation is as follows.

Each blocking molecule comprised in the binding-blocking molecule unit may comprise any one of amino acid sequences as set forth in SEQ ID NOs: 7 to 9 and 16 to 19 or comprises an amino acid sequence that has at least 50%, 60%, 70, 80%, 85%, 90%, 95% or 99% identity to any one of SEQ ID NOs: 7 to 9 and 16 to 19.

For example, the blocking molecule comprises an amino acid sequence that is identical to SEQ ID No: 7. Alternatively, for another example, the blocking molecule has an amino acid sequence that has at least 50%, 60%, 70, 80%, 85%, 90%, 95% or 99% identity to SEQ ID NO: 7.

SEQ ID NOs: 16 to 19 are specifically shown as follows:

SEQ ID NO: 16 (Pep170):

STIEEQAKTFLDKFNHEAEDLFYQSSLASWNYNTNITEENVQNMNNAGDKWSAFLK
EQSTLAQMYPLQEIQNLTVKLGGGGSGGGGSTSGGVTGGLPNMTQGFWENSMLTDP
GNVQKAVCHPTAWDLGKGDFRILMCTKVTMDDFLTAHHEMGHIQYDMAYAAQPF
LLRN;

SEQ ID NO: 17 (Pep180):

STIEEQAKTFLDKFNHEAEDLFYQSSLASWNYNTNITEENVQNMNNAGDKWSAFLK
EQSTLAQMYPLQEIQNLTVKLQLQALGGGGSGGGGSTSGGVTGFFVSVGLPNMTQG
FWENSMLTDPGNVQKAVCHPTAWDLGKGDFRILMCTKVTMDDFLTAHHEMGHIQ
YDMAYAAQPFLLRN;

SEQ ID NO: 18 (Pep190):

STIEEQAKTFLDKFNHEAEDLFYQSSLASWNYNTNITEENVQNMNNAGDKWSAFLK
EQSTLAQMYPLQEIQNLTVKLQLQALQQGGGGSGGGGSTSGGVTGRIFKEAEKFFVS
VGLPNMTQGFWENSMLTDPGNVQKAVCHPTAWDLGKGDFRILMCTKVTMDDFLT
AHHEMGHIQYDMAYAAQPFLLRN;

SEQ ID NO: 19 (Pep200):

STIEEQAKTFLDKFNHEAEDLFYQSSLASWNYNTNITEENVQNMNNAGDKWSAFLK
EQSTLAQMYPLQEIQNLTVKLQLQALQQGGGGSGGGGSTSGGVTGGHAGGTVDAQ
RIFKEAEKFFVSVGLPNMTQGFWENSMLTDPGNVQKAVCHPTAWDLGKGDFRILM
CTKVTMDDFLTAHHEMGHIQYDMAYAAQPFLLRN.

In an illustrative example, the blocking molecule may be a peptide fragment or an antibody. When the antibody, preferably a nanobody, is used, the size of the monomer structure as a whole can be reduced.

When blocked by a blocking peptide, compared with antibodies, the entire structure has a smaller size and is a simpler structure, which is more suitable for obtaining by means of expression in cells, secretion from cells, or in-vitro expression. Specifically, by using the blocking peptide fragment, the entire monomer structure has a size in a range from 30 to 80 KD.

When there is a plurality of blocking peptide fragments, the possibility of blocking the binding between the virus and the cell receptor can be increased, thereby improving the above-mentioned blocking effect.

In an illustrative example, the cell receptor is ACE2.

In an illustrative example, the binding-blocking molecule unit comprises at least one first blocking molecule (a first blocking peptide fragment, denoted by B 1 in order to facilitate illustration) and/or at least one second blocking molecule (a second blocking peptide fragment, denoted by B2 in order to facilitate illustration). The first blocking molecule comprises an amino acid sequence that is identical to SEQ ID NO: 7 or has at least 50%, 60%, 70, 80%, 85%, 90%, 95% or 99% identity to SEQ ID NO: 7, and the second blocking molecule comprises an amino acid sequence that is identical to any one of SEQ ID NOs: 8 and 16 to 19 or has at least 50%, 60%, 70, 80%, 85%, 90%, 95% or 99% identity to any one of SEQ ID Nos: 8 and 16 to 19. The first blocking molecule and/or the second blocking molecule are respectively peptide fragments, which have a superior blocking effect on binding between the virus and the cell receptor over a nanobody.

In an illustrative example, the binding-blocking molecule unit comprises two first blocking molecules or two second blocking molecules. More preferably, the two first blocking molecules or the two second blocking molecules are linked to each other respectively via a N-terminus of one to a C-terminus of the other, i.e., B1-B1 or B2-B2.

In an illustrative example, when the binding-blocking molecule comprises the first blocking molecule and the second blocking molecule, the first blocking molecule is linked to the second blocking molecule by linking the N-terminus of one to the C-terminus of the other. That is, the N-terminus of the first blocking molecule is linked to the C-terminus of the second blocking molecule, or the N-terminus of the second blocking molecule is linked to the N-terminus of the first blocking molecule.

Preferably, the N-terminus of the second blocking molecule is linked to the C-terminus of the second blocking molecule. In this case, the monomer structure has a structure, for example, as shown in Formula 3 or Formula 4:

B1-B2-A (Formula 3). Preferably, the structure is linked in a manner that the C-terminus of B 1 is linked to the N-terminus of B2, the C-terminus of B2 is linked to the N-terminus of A, that is, linked to the C-terminus of the former and to the N-terminus of the latter (same below).

B 1-B2-D-A (Formula 4). Preferably, the structure is linked in the following manner that the C-terminus of B1 is linked to the N-terminus of B2, the C-terminus of B2 is linked to the N-terminus of D, and the C-terminus of D is linked to the N-terminus of A.

In an illustrative example, the monomer structure further comprises a leading peptide (denoted by C in order to facilitate illustration) that is configured to promote the production of the polymer structure by expression in the cells or secretion from the cells, or by in vitro cell-free expression. The leading peptide comprises an amino acid sequence that is identical to SEQ ID NO: 10 or has at least 50%, 60%, 70, 80%, 85%, 90%, 95% or 99% identity to SEQ ID NO: 10. In such a case, the leading peptide, in addition to the binding-blocking molecule unit and polymerizing molecule, is comprised in the monomer structure. For example, the monomer structure has a structure shown in Formula 5:

C-B-A (Formula 5). Preferably, the C-terminus of C is linked to the N-terminus of B, and the C-terminus of B is linked to the N-terminus of A.

When there are two blocking molecules, preferably, the leading peptide is linked to an N-terminus of either blocking molecule. In this case, the monomer structure has a structure shown, for example, in Formula 6:

C-B1-B2-A (Formula 6). The structure is linked preferably in a manner that, the C-terminus of C is linked to the N-terminus of B1, the C-terminus of B1 is linked to the N-terminus of B2, and the C-terminus of B2 is linked to the N-terminus of A.

When there is a linker molecule, the monomer structure has a structure shown in Formula 7:

C-B1-B2-D-A (Formula 7). Preferably, the structure is linked, preferably in a manner that the C-terminus of the former is linked to an N-terminus of the latter. That is, the C-terminus of C is linked to an N-terminus of B1, the C-terminus of B1 is linked to an N-short chain of B2, the C-terminus of B2 is linked to the N-terminus of D, and the C-terminus of D is linked to the N-terminus of A.

In an illustrative example, the acidic structure comprises at least two consecutive aspartates (aspartates denoted by d in order to facilitate illustration), and/or at least two consecutive glutamates (glutamates denoted by e in order to facilitate illustration), and/or at least a combination of aspartates and amino acids. For example, the acidic structure comprises 10 consecutive aspartates (denoted by 10d), 10 consecutive glutamates (denoted by 10e), 8 consecutive aspartates and 8 consecutive glutamates (denoted by 8d8e), or 5 d-e (5de, one d and one e are in one group, 5 groups in total).

In an illustrative example, the stabilizing molecule is linked to the C-terminus of the polymerizing molecule. In such case, the monomer structure has a basic structure shown in Formula 8.

B-A-E (Formula 7). In this formula, preferably, the C-terminus of the former is linked to the N-terminus of the latter.

Based on this, in combination with any one or more of the foregoing C and D, different formulas, for example, Formula 9 to Formula 11, can be derived.

C-B-A-E (Formula 9). In this formula, preferably, the C-terminus of the former is linked to the NC-terminus of the latter.

B-D-A-E (Formula 10). In this formula, preferably, the C-terminus of the former is linked to the N-terminus of the latter.

C-B-D-A-E (Formula 11). In this formula, preferably, the C-terminus of the former is linked to the N-terminus of the latter.

When B includes B1 and B2, for example, it can be shown in Formula 12.

C-B1-B2-D-A-E (Formula 12). In this formula, preferably, the C-terminus of the former is linked to the N-terminus of the latter.

In an illustrative example, the monomer structure further comprises a protein tag (denoted by F in order to facilitate illustration) that is configured to promote the purification of the polymer structure or the polymer structure. In an illustrative example, the protein tag comprises an amino acid sequence that is identical to SEQ ID NO: 15 or has at least 50%, 60%, 70, 80%, 85%, 90%, 95% or 99% identity to SEQ ID NO: 15.

Preferably, the protein tag has a C-terminus that is linked to the N-terminus of the blocking-binding molecule unit, that is, the C-terminus of the protein tag is located at the N-terminus of the blocking-binding molecule unit. In this case, the monomer structure has a basic structure shown in Formula 13.

F-B-A (Formula 13). In this formula, preferably, the C-terminus of the former is linked to the N-terminus of the latter.

Based on this, in combination with any one or more of the foregoing C, D, and E, different formulas, for example, Formula 14 to Formula 16, can be derived.

F-B-D-A (Formula 14). In this formula, preferably, the C-terminus of the former is linked to the N-terminus of the latter.

C-F-B-A (Formula 15). In this formula, preferably, the C-terminus of the former is linked to the N-terminus of the latter.

F-C-B-D (Formula 16). In this formula, preferably, the C-terminus of the former is linked to the N-terminus of the latter. In such a case, although the C-terminus of F is not directly linked to the N-terminus of B, but via C, that is, the C-terminus of F is located at the N-terminus of B (other similar explanation).

In an illustrative example, when the monomer structure further comprises the leading peptide, the leading peptide is linked to the binding-blocking molecule unit via the protein tag, as shown in Formula 17.

C-F-B-D-A (Formula 17). Preferably, the C-terminus of the former is linked to the N-terminus of the latter. Similarly, in such a case, the leading peptide is not directly linked to the binding-blocking molecule unit, but via the protein tag.

In the foregoing different formulas, when the blocking peptide fragment is B 1 and/or B2, B 1 and B2 occur at the same time, where in the foregoing formula, the positions of B1 and B2 may be B1-B2, or may be B2-B1; alternatively, only either one may occur, wherein B is denoted by B 1 or B2.

In an illustrative example, the above-mentioned virus is any one or more of hepatitis B virus, rabies virus, HVP, and COVID-19 virus.

In an illustrative example, the monomer structure involved in this example has a size in a range from 30 to 80 KD.

In an illustrative example, the binding-blocking structure unit has a binding force that is 1000 to 1000,000 times greater than a nanobody.

In an illustrative example, the polymer structure is soluble, for example in an aqueous solution.

In an illustrative example, when the monomer structures are polymerized through the polymerizing molecule, the polymer structure is thermally stable at a temperature that is closer to 70°C, preferably, greater than or equal to 80°C.

This example further provides a nucleic acid encoding the foregoing monomer structure or the foregoing polymer structure.

This example further provides a vector comprising the above-mentioned nucleic acid.

This example further provides a eukaryotic host cell that comprising the above-mentioned nucleic acid or vector for the expression in cells, secretion from cells or in vitro cell-free synthesis and expression of the above-mentioned monomer structure or the above-mentioned polymer structure.

This example further provides a use of the above-mentioned nucleic acid or the above-mentioned vector in a method for preparing the monomer structure and/or the polymer structure.

Due to the blocking advantage brought by the polymer structure formed by polymerizing the monomer structure through the above polymerizing molecule, this example can have a better blocking function in the following applications or products, so as to better prevent, treat, or detect (or other actions) a specific virus.

(1) This example further provides a use of any one of the above-mentioned polymerizing molecule, the above-mentioned monomer structure, and the above-mentioned polymer structure for: a medicament for treating a virus, detection and diagnosis of a virus, a medical use, a disinfection product against a virus, a cosmetic product, a skin care product, a care product, food, or a cleaning product.

(2) This example further provides a disinfection product, a cosmetic product, a skin care product, a care product, a food or a cleaning product, characterized in that, comprising one or more of any one of the above-mentioned polymerizing molecule, the above-mentioned monomer structure, or the above-mentioned polymer structure.

(3) This example further provides a medicament comprising: one or more of the above-mentioned polymerizing molecule, the above-mentioned monomer structure, and the above-mentioned polymer structure; and a pharmaceutically acceptable carrier, diluent, or excipient.

The above-mentioned disinfection products can be used for, for example, air disinfection, water disinfection, food disinfection, clothing disinfection, or can be used in any position, situations, or place that may need to be disinfected such as household furniture, and in various tools such as a vehicle or facilities such as a bus station.

The skin care product can be a product, for example, a facial cream, a toning spray, a toning water, an eye cream, or the like to be coated on a skin surface.

The above-mentioned cosmetic product can be, for example, a foundation makeup, a cheek color, or the like.

The above-mentioned care product can be, for example, a care solution, a care ointment, a spray, or the like, such as an eye drop, or auxiliary product, such as a contact lens care solution, for a susceptible virus-infected surface or auxiliary product.

The above-mentioned cleaning product can be, for example, a detergent, a shampoo, a bath lotion, a liquid detergent, or the like.

The polymer structure herein is also referred to as a virus blocking protein or a blocking protein.

Preferably, the blocking protein is produced by KANGMA-HEATHCODE (SHANGHAI) BIOTECH CO., LTD., using a D2P technology, such as a D2P system. The D2P technology, for example, comprises cloning an encoding optimized gene into a pD2P vector. The plasmid was amplified by using an Ampi system and then added to the Protein Factory Rapid Reaction System (from KANGMA-HEATHCODE (SHANGHAI) BIOTECH CO., LTD.) with a volume ratio of 1: 30. The reaction mixture was incubated at 30°C for 4 hours and then collected and purified by centrifugation. The supernatant of the cell-free mixture was vortexed with His Monster Magnetic Beads (KANGMA-HEATHCODE (SHANGHAI) BIOTECH CO., LTD.) for 1 hour at 4°C. A washing buffer (50 mM Tris-HCl, pH 8.0, 500 mM NaCl, and 10 mM imidazole) and an elution buffer (50 mM Tris-HCl, pH 8.0, 500 mM NaCl, and 250 mM imidazole) were used.

The Ampi system is: a random primer with a final concentration of 20 to 30 $\mu$M, a plasmid template of 0.05 to 0.15 $\mu$g/mL, dNTP of 0.5 to 1 mM, 2 x BSA, phi29DNA polymerase of 0.05 to 0.1 mg/mL, and 1 x phi29 reaction buffer (50 mM Tris-HCl, 10 mM MgCl$_2$, 10 mM (NH$_4$)$_2$SO$_4$, and 4 mM DTT, pH7 .5).

Example 2

In this example, a specific experiment in which a COVID-19 virus as a virus and an ACE2 as a cell receptor are used were carried out, to describe blocking advantages of the various polymer structures.

1. IVTT reaction for Preparation of the virus-blocking protein involved in the example.

The gene sequences of the optimized target protein (including the gene sequences encoding the above mentions various virus-blocking protein structures) were inserted into the plasmid, and then introduced into the homemade Kluyveromyces lactis in vitro cell-free protein synthesis system, which was prepared with Kluyveromyces lactis (KluyveromyceslactisNRRL Y-1140) in vitro. The in vitro cell-free protein synthesis system (having a total volume of 30 $\mu$L) used in this example comprises 50%(v/v) Kluyveromyces lactis cell extract, 22 mM tri(hydroxymethyl)aminomethane (pH8), 90 mM potassium acetate, 4.0 mM magnesium acetate, 3.0 mM nucleoside triphosphate mixture, 0.16 mM amino acid mixture, 22 mM potassium phosphate, 0.003 mg/mL amylase, 3% (w/v) polyethylene glycol (PEG-8000), 340 mM maltodextrin (in glucose units, equal to about 55mg/mL), 0.04 mg/mL exogenous RNA, 15 ng/$\mu$L DNA of the target protein, and the like. When more than one type of the fluorescent protein DNA is used, 15 ng/$\mu$L herein is the total concentration of DNA for the respective fluorescent proteins. The reaction system was placed in an environment of 22 to 30°C and incubated at rest for about 20 hours.

After IVTT reaction, His Magnetic Beads (a biological product from KANGMA-HEATHCODE (SHANGHAI) BIOTECH CO., LTD.) were used for purification. After protein elution, it was subjected to ultrafiltration and centrifugation, and was replaced with PBS buffer. The purified protein was obtained after filtering through a 0.22 $\mu$m syringe filter, and stored at 4 °C for later use.

1. the structures of the prepared virus-blocking proteins involved in the various experiments in this example are described as follows.

| Table 2 | | |
|---|---|---|
| No. | Name of constructed structures | structure specification (from N-terminal to C-terminal) |
| 1 | Kmds001 | C-F-B1-B2-D1-A: Leading-Histag-Pep56-Pep160-Tram-OctaTag |
| 2 | Kmds002 | C-F-B1-B2-D1-A-E (Kmds001+acidic structure E10d): Leading-Histag-Pep56-Pep160-Tram-OctaTag-MrTail (the acidic structure has a sequence of 10d) |
| 3 | Kmds003 | C-F-B1-B2-D1-A-E (Kmds001+acidic structure E10e): Leading-Histag-Pep56-Pep160-Tram-OctaTag-MrTail (the acidic structure has a sequence of 10e) |
| 4 | Kmds006 | C-F-B1-B2-D1-A-E (Kmds001+ sequence of the acidic structure E8d8e): Leading-Histag-Pep56-Pep160-Tram-OctaTag-MrTail (the acidic structure has a sequence of E8d8e) |
| 5 | Kmds008 | C-F-B1-D1-A (Kmds001- B2): Leading-Histag-Pep56-Tram-OctaTag |
| 6 | Kmds007 | F-B2-D1-A (Kmds001- B1): Leading-Histag-Pep160-Tram-OctaTag |
| 7 | Kmds011 | C-F-B2-D1-A-E (Kmds007+acidic structure E10d): Leading-Histag-Pep160-Tram-OctaTag-MrTail (the acidic structure has a sequence of E10d) |
| 8 | Kmds012 | C-F-B2-D1-A-E (Kmds007+acidic structure E10e): Leading-Histag-Pep160-Tram-OctaTag-MrTail |
| 9 | Kmds013 | F-B2-D1-A-E (Kmds007+acidic structure E5de): Leading-Histag-Pep160-Tram-OctaTag-MrTail (the acidic structure has a sequence of E5de) |

(continued)

| | Table 2 | |
|---|---|---|
| No. | Name of constructed structures | structure specification (from N-terminal to C-terminal) |
| 10 | Kmds0014 | C-F-B2-D1-A-E (Kmds007+acidic structureE8d8e): Leading-Histag-Pep160-Tram-OctaTag-MrTail (the acidic structure has a sequence of E8d8e) |
| 11 | Kmds009-2 | C-F-B3-D1-A (Kmds007 with substituting Pep160 with Pep130) Leading-Histag-Pep130-Tram-OctaTag |
| 12 | Kmds009 | F-B3-D1 Leading-Histag-Pep130-Tram |
| 13 | Kmds044 | C-F-B2-B2-D1-A-E (Kmds012 with adding one B2): Leading-Histag-Pep160-Pep160-Tram-OctaTag-MrTail |
| 14 | KMds030 | F-B1-B2-D1-A-E (KMds003-leading peptide C) Histag-Pep56-Pep160-Tram-OctaTag-MrTail |
| 15 | KMds031 | F-B1-B2-D1-A-E (KMds006-leading peptide C) Histag-Pep56-Pep160-Tram-OctaTag-MrTail |
| 16 | KMds032 | F-B2-D-A-E (Kmds0012-leading peptide C) Leading-Histag-Pep56-Pep160-Tram-OctaTag-MrTail |
| 17 | KMds033 | F-B2-D1-A-E (Kmds014-leading peptide C) Histag-Pep160-Tram-OctaTaa-MrTail |
| 18 | KMds104 | F-B2-B2-D1-A-E (Kmds044-leading peptide C) Histag-Pep160-Pep160-Tram-OctaTaa-MrTail |
| 19 | KMds036 | C-F-B1-B2-A-E (KMds003-linker molecule D): Leading-Histag-Pep56-Pep160-OctaTag-MrTail |
| 20 | KMds038 | C-F-B2-A-E (Kmds012- linker molecule D): Leading-Histag-Pep160-OctaTag-MrTail |
| 21 | KMds108 | C-F-B2-A-E (Kmds007-D): Leading-Histag-Pep160-Pep160-OctaTag-MrTail |
| 22 | KMds045 | C-F-B1-B2-D2-A-E, obtained by deleting a part of Tram but reserving the 10 amino acid residues at C-terminus of Tram in KMds003: Leading-Histag-Pep56-Pep160-Tram without the deleted portion - OctaTag-MrTail (the acidic structure has a sequence of 10e) |
| 23 | KMds046 | C-F-B2-D2-A-E, obtained by deleting a part of Tram but reserving the 10 amino acid residues at C-terminus of Tram in KMds012: Leading-Histag-Pep160-Tram without the deleted portion - OctaTag-MrTail |
| 24 | KMds109 | B2-B2-D2-A-E: obtained by deleting a part of Tram but reserving the 10 amino acid residues at C-terminus of Tram in KMds104: Leading-Histag-Pep160-Pep 160-Tram without the deleted portion - OctaTag-MrTail |
| 25 | KMds042 | C-F-B1-B2-D1-E (Kmds003-polymerizing molecule A): obtained by deleting OctaTag from Kmds003, Leading-Histag-Pep56-Pep160-Tram-MrTail |
| 26 | KMds043 | C-F-B2-D1-E (KMds012- polymerizing molecule A): Leading-Histag-Pep160-Tram-MrTail |

(continued)

| Table 2 | | |
|---|---|---|
| No. | Name of constructed structures | structure specification (from N-terminal to C-terminal) |
| 27 | KMds105 | F-B2-B2-D3-A-E, obtained by substituting Tram in KMds104 with Fc: Leading-Histag-Pep160-Pep160-Fc-OctaTag-MrTail |
| 28 | KMds121 | F-B2-B2-D4-A-E, obtained by substituting Tram in KMds104 with a residue sequence that form α-helix in HSA: Leading-Histag-Pep160-Pep160-HSA-OctaTag-MrTail |
| 29 | KMds112 | F-B2-D5-A-E: obtained by substituting Tram in KMds032 with a residue sequence that form α-helix in HSA, and substituting Cys in the residue sequence with Gly: Leading-Histag-Pep56-Pep160-HSA-OctaTag-MrTail |
| 30 | Kmds052 | C-F-B1-B2-E (Kmds003-A-D) Leading-Histag-Pep56-Pep160-MrTail (the acidic structure has a sequence of 10e) |
| In this table, "+" represents adding and "-" represents deleting | | |

The sequences involved in Table 2 are illustrated in Table 3:

| Table 3 | | |
|---|---|---|
| Name Code | SEQ ID NO. | Amino acid sequence |
| A: polymerizing molecule OctaTag | SEQ ID NO: 1 | SDVQSSLTGTWYNELNSKMELTANKDGTLTGKYL SKVGDVYVPYPLSGRYNLQPPAGQGVALGWAVS WENSKIHSATTWSGQFFSESSPVILTQWLLSSSTAR GDVWESTLVGNDSFTKTAPTEQQIAHAQLHCRAP RLK |
| D1 linker molecule: EGFP | SEQ ID NO: 2 | VSKGEELFTGVVPILVELDGDVNGHKFSVRGEGE GDATNGKLTLKFICTTGKLPVPWPTLVTTLTYGVQ CFSRYPDHMKQHDFFKSAMPEGYVQERTISFKDD GTYKTRAEVKFEGDTLVNRIELKGIDFKEDGNILG HKLEYNFNSHNVYITADKQKNGIKANFKIRHNVE DGSVQLADHYQQNTPIGDGPVLLPDNHYLSTQSK LSKDPNEKRDHMVLLEFVTAAGITLGMDELYK |
| D2 linker molecule obtained by deleting a part of D1 but reserving 10 amino acids at its C-terminal. | SEQ ID NO: 3 | GITLGMDELYK |
| D3 linker molecule: Fc of IgG4 | SEQ ID NO: 4 | PSVFLFPPKPKDTLMISRTPEVTCVVVDVSQEDPE VQFNWYVDGVEVHNAKTKPREEQFNSTYRVVSV LTVLHQDWLNGKEYKCKVSNKGLPSSIEKTISKA KGQPREPQVYTLPPSQEEMTKNQVSLTCLVKGFY PSDIAVEWESNGQPENNYKTTPPVLDSDGSFFLYS RLTVDKSRWQEGNVFSCSVMHEALHNHYTQKSL SLSLGK |
| D4 linker molecule: a part of HAS, which is a residue sequence that forms α-helix in HSA | SEQ ID NO: 5 | ADDKETCFAEEGKKLVAASQAA |

(continued)

| Table 3 | | |
|---|---|---|
| Name Code | SEQ ID NO. | Amino acid sequence |
| D5 linker molecule: a part of HAS, which is a residue sequence that forms $\alpha$-helix in HSA and having Cys in the residue sequence substituted with Gly | SEQ ID NO: 6 | ADDKETGFAEEGKKLVAASQAA |
| B1: blocking molecule Pep56 | SEQ ID NO: 7 | DKEWILQKIYEIMRLLDELGHAEASMRVSDLIYEF MKKGDERLLEEAERLLEEVER |
| B2: blocking molecule Pep160 | SEQ ID NO: 8 | STIEEQAKTFLDKFNHEAEDLFYQSSLASWNYNT NITEENVQNMNNAGDKWSAFLKEQSTLAQMYPL QEIQNGGGGSGGGGSTSGGVTGTQGFWENSMLT DPGNVQKAVCHPTAWDLGKGDFRILMCTKVTMD DFLTAHHEMGHIQYDMAYAAQPFLLRN |
| B3: blocking molecule Pep130 | SEQ ID NO: 9 | PSVFLFPPKPKDTLMISRTPEVTCVVVDVSQEDPE VQFNWYVDGVEVHNAKTKPREEQFNSTYRVVSV LTVLHQDWLNGKEYKCKVSNKGLPSSIEKTISKA KGQPREPQVYTLPPSQEEMTKNQVSLTCLVKGFY PSDIAVEWESNGQPENNYKTTPPVLDSDGSFFLYS RLTVDKSRWQEGNVFSCSVMHEALHNHYTQKSL SLSLGK |
| C: leading peptide | SEQ ID NO: 10 | ITETSSPFRSIFSHSGK |
| E: acidic structure (amino acid tail) 10d | SEQ ID NO: 11 | DDDDDDDDDD |
| E: acidic structure (amino acid tail) 10e | SEQ ID NO: 12 | EEEEEEEEEE |
| E: acidic structure (amino acid tail) 5de | SEQ ID NO: 13 | DEDEDEDEDE |
| E: acidic structure (amino acid tail) 8d8e | SEQ ID NO: 14 | DDDDDDDDEEEEEEEE |
| F: protein tag | SEQ ID NO: 15 | HHHHHHHH |

2. pseudovirus and cell pre-test

The S protein was expressed in the surface of in the COVID-19 pseudovirus used and the luciferase gene was encapsulated therein. After cell infection, luciferase proteins can be expressed in cells, and the efficiency of pseudovirus infecting cells can be obtained by adding a substrate of luciferase and detecting the luminous value of the substrate for the luciferase.

First, the cell infection by the pseudovirus was pre-tested to determine the dilution of the pseudovirus. The pseudovirus was serially diluted at a gradient of 30 times (see Table 4 for details) and infected HEK293T or HEK293T-ACE2 (ACE2-overexpressing HEK293T cells), respectively. Experiments were carried out in duplicate for each infection.

Finally, a linear window detected with luciferase was found to be in the range of S3-S7 (270 to 21870-fold dilution) when the cells were infected by virus. The pseudovirus supplier recommends using a virus at 540-fold dilution to infect cells, so we subsequently used a pseudovirus at 540-fold dilution.

In the experiment of infection of wild-type cells and ACE2-overexpressing HEK293T cells with wild-type COVID-19 strains, it was found that the efficiency of virus infection in ACE2-overexpressing cells was significantly higher than that in wild-type cells. Therefore, in subsequent experiments, ACE2-overexpressing HEK293T cells were selected as the cell strain to be tested.

| Table 4 | | |
|---|---|---|
| concentration (mg/ml) | eGFP | Kmds001 |
| C1 | 1.298767 | 1.169667 |
| C2 | 0.432922 | 0.389889 |
| C3 | 0.144307 | 0.129963 |
| C4 | 0.048102 | 0.043321 |
| C5 | 0.016034 | 0.014440 |
| C6 | 0.005345 | 0.004813 |
| C7 | 0.001782 | 0.001604 |
| C8 | 0.000594 | 0.000535 |
| C9 | 0.000198 | 0.000178 |
| C10 | 0.000066 | 0.000059 |
| C11 | 0.000022 | 0.000020 |
| C12 | 0.000007 | 0.000007 |

3. Kd evaluation

Surface Plasma Resonance (SPR) Experiment

SPR Research was carried out using a Biacore T200 Biosensor (GE Healthcare, USA). The virus-blocking protein was fixed to a S-series sensor chip CM5 (GE Healthcare, USA) by amine coupling. A Reference strain for His-tagged RBD and an Omicron variant (Sino Biological Inc, Beijing, China) were diluted at six concentrations (0.25, 0.5, 1, 2, 4 and 8 nM), and RBD of a Delta variant (Sino Biological Inc, Beijing, China) was diluted at (0.5, 1, 2, 4, 8 and 16 nM). Single cycle dynamics analysis was carried out at 25°C. In 10 mM HEPES, pH 7.4, 150 mM NaCl, 3 mM EDTA and 0.05% Tween, the association phase was set at 120 s and 30 μL/min, and the dissociation phase was set at 300 s and 30 μL/min respectively, and - 20 as the running buffer. The analysis repeated nine times. The concentrations of the analyte were arranged in ascending order. The value of dissociation constant (Kd) was calculated using a Biacore T200 evaluation software (version 2.0, GE Healthcare, USA).

The results show that the structures provided in Table 2 of the present disclosure strongly interacted with the RBD domains of all three virus strains, i.e., have strong binding affinity to the original strain, Delta and Omicron variants. The values of the dissociation constant (Kd) for the original strain, Delta and Omicron variants were 1.25 nM, 0.837 nM and 0.656 nM, respectively.

In summary, the virus-blocking proteins of the present disclosure are still qualified for binding to the RBD domain of the Omicron variant, although the Omicron variant has higher health risks and a surprising immune escape.

4. Procedures in the experiment in which the pseudovirus was blocked by different structures provided in Table 2 are as follows:

(1) HEK293T-ACE2 cells to be infected were inoculated into 48-well cell culture plates at an amount of $1.5 \times 10^4$ cells per well. The cell density was about 30% when the pseudovirus infection test was performed the next day.

(2) The next day, SARS-CoV-2 (2019-nCoV) S protein pseudovirus was took out from -80°C and thawed on ice or thawed spontaneously at 4°C. After complete thawing, the pseudovirus was 270-fold diluted with complete culture medium (DMEM, 10% fetal bovine serum, 1% two antibiotics, 0.75 μg/mL purmatomycin) to obtain pseudovirus diluent.

(3) The KMds blocker, which has an original concentration of 1 μM, was serially diluted with complete culture medium at a gradient of 10 times. The gradiently diluted KMds blockers was mixed with the pseudovirus diluent at a volume ratio of 1: 1 to form a pseudovirus infection solution. The pseudovirus infection solution was incubated at room temperature for 1 hour.

(4) One hour later, the 48-well plate with HEK293T- ACE2 cells pre-spreaded from the incubator was took out. After the density and state of the cells were confirmed, and the upper culture medium was pipetted away. 200 μL pseudovirus infection solution was added to each well along the wall of the well to avoid cell flushing. The 48-well plate was incubated in an incubator for 6 hours.

(5) After 6 hours, the pseudovirus infection solution was carefully pipetted, replaced into 300 μL fresh complete culture medium, and continued to culture in the incubator for 48 hours.

(6) After 48 hours, the culture medium was pipetted and 200 μL 1 × PBS was carefully added to each well to wash the cells while avoiding cell flushing. PBS was carefully pipetted, 65 μL 1 × cell lysate was added to each well, and incubated at room temperature for 15 to 20 minutes. Immediately after 15-20 minutes, the luciferase activity was detected or the cell lysate was stored at -20°C.

(7) 5 μL cell lysate was mixed with 5 μL luciferase substrate (Promega E1501 luciferase Assay system), and the mixture was added to the 384-well plate. The luciferase activity was immediately detected with the Perkin Elmer Envision 2102 multimode plate reader, and the inhibition efficiency of the KMds blocker was determined.

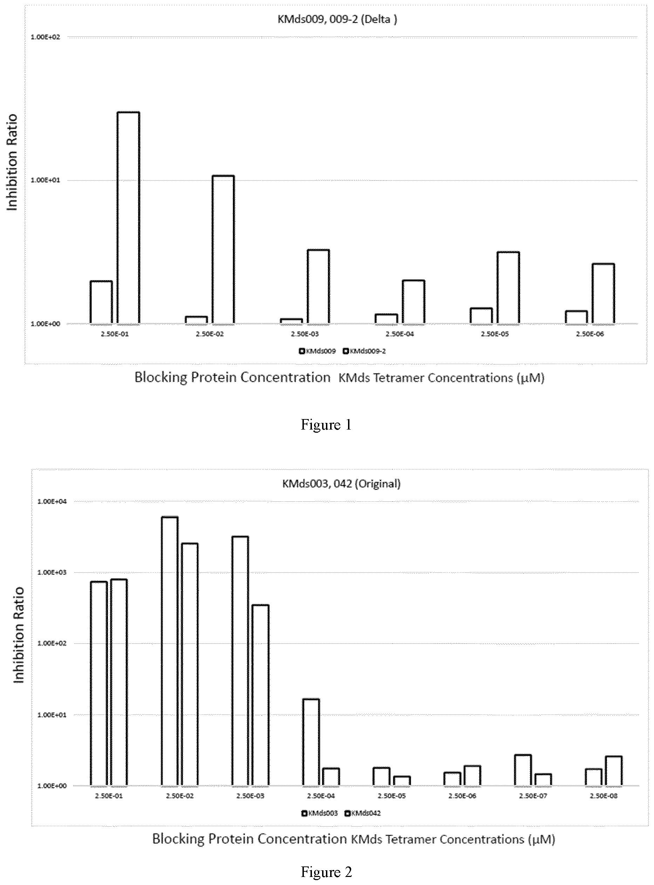

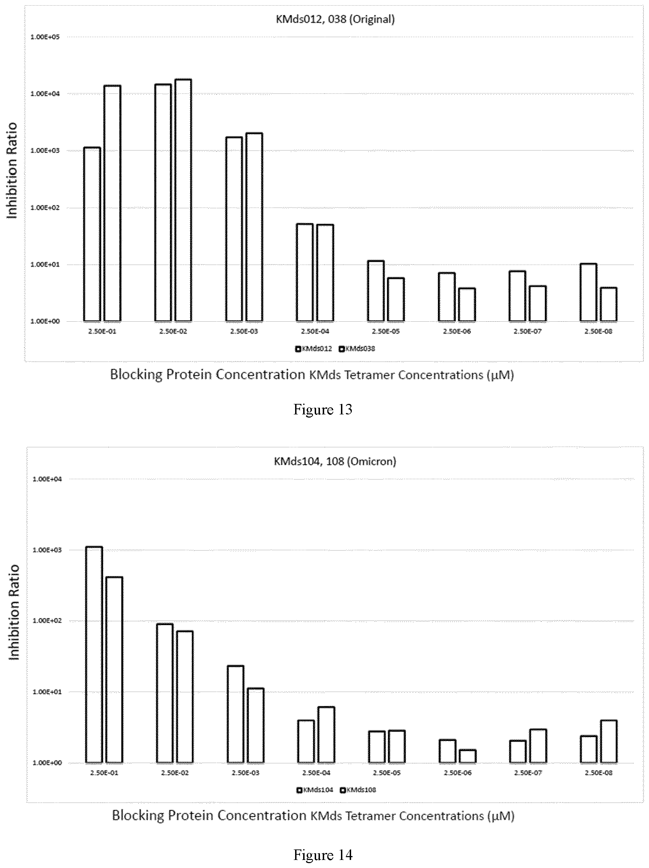

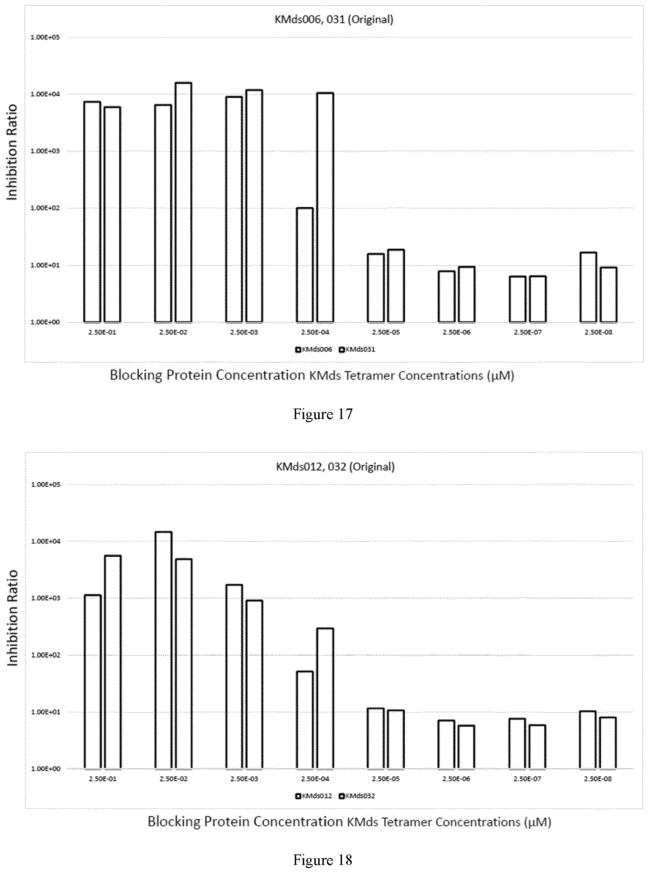

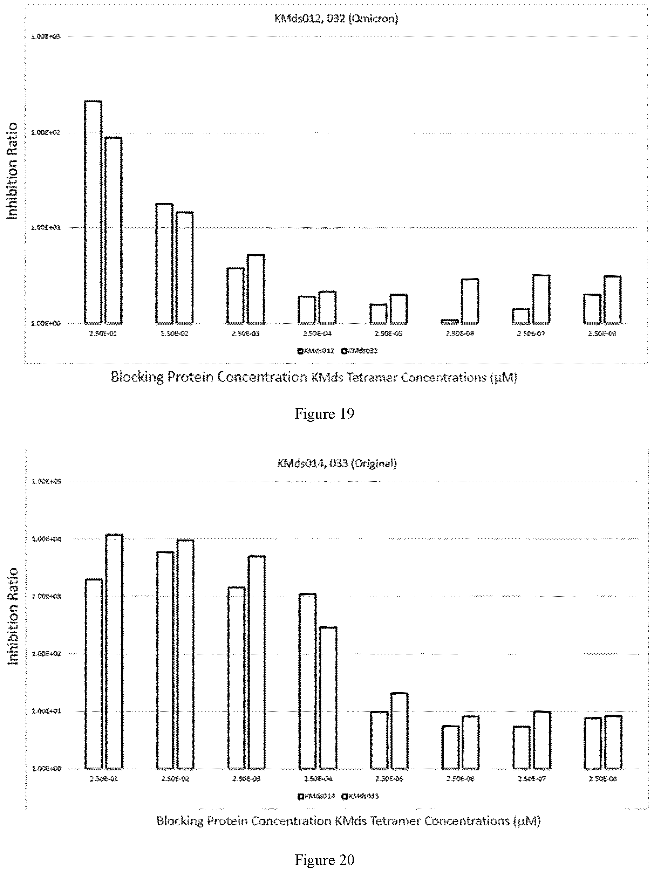

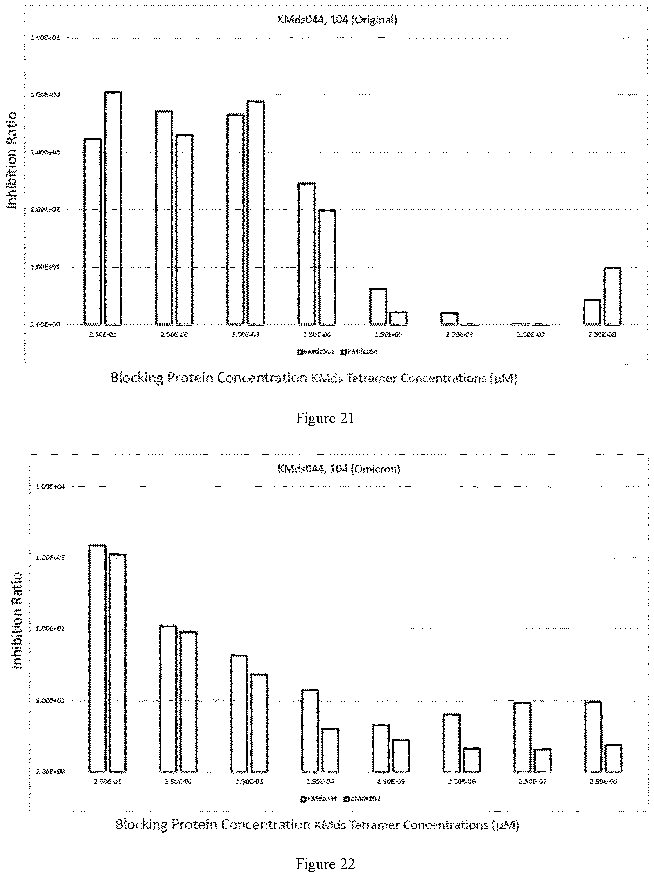

The test results are shown in FIGs. 1 to 23. The horizontal coordinate in the figure represents a concentration of a virus-blocking protein used; the vertical coordinates in the figure represents the inhibition effect (inhibition rate) after blocking test. The inhibition rate is calculated as follows:

$$\text{The inhibition rate of virus} - \text{blocking protein} = \frac{V0}{V1}$$

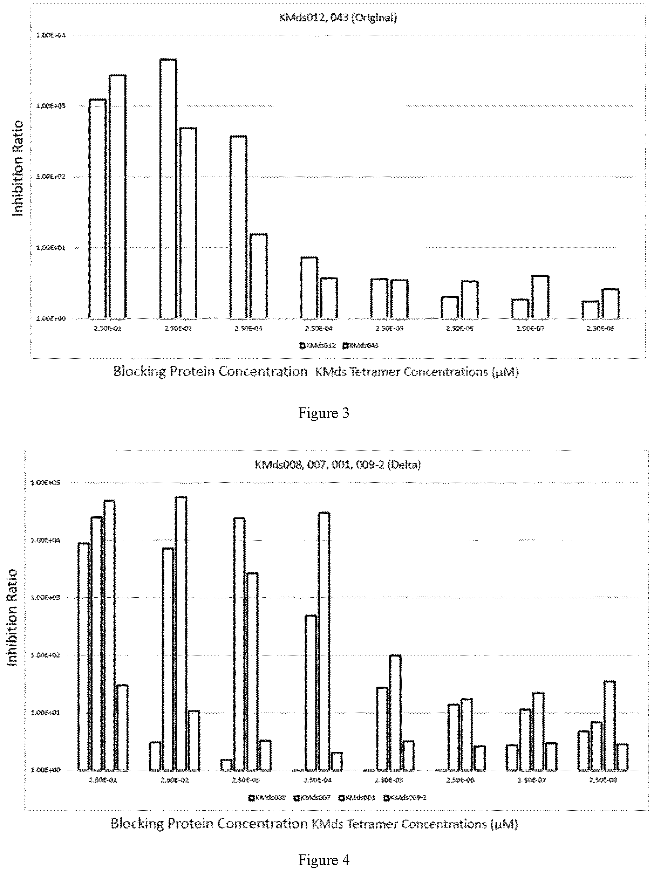

V0 is an RLU reading of a control where only a virus was added; V1 is an RLU reading of a sample with a virus-blocking protein and a virus.
The higher the vertical coordinate value, the better the result.
Delta indicates an inhibition test for a Delta pseudovirus, Original indicates an inhibition test for COVID-19 wild-type pseudovirus, and Omicron indicates an inhibition test for Omicron pseudovirus).
Each group of bars in the column diagrams in figure in a sequence from left to right corresponds to a diagram of results for a structure of which the name is displayed in the legend or the title. For example, in FIG. 4, the legend shows Kmds008, Kmds007, Kmds001, and Kmds009-2 in a sequence from left to right, and the sequence of each group of bars in the column diagram also corresponds to a result of a corresponding structure to this sequence.
The results are as follows:

I. Effect before and after polymerization:
As shown in FIGs. 1-3, the effect before polymerization (Kmds009) is worse than that after polymerization (Kmds009-2). The effect before polymerization (Kmds042) is worse than that after polymerization (Kmds003). The effect before polymerization (Kmds043) is worse than that after polymerization (Kmds012).

II. Blocking effects of different blocking molecules. The test shows, the blocking molecules of the present disclosure have a specific binding capability to the S protein of the COVID-19 virus.

(1) As shown in FIG. 4, different blocking molecules at lower concentrations can inhibit COVID-19 and Delta virus.

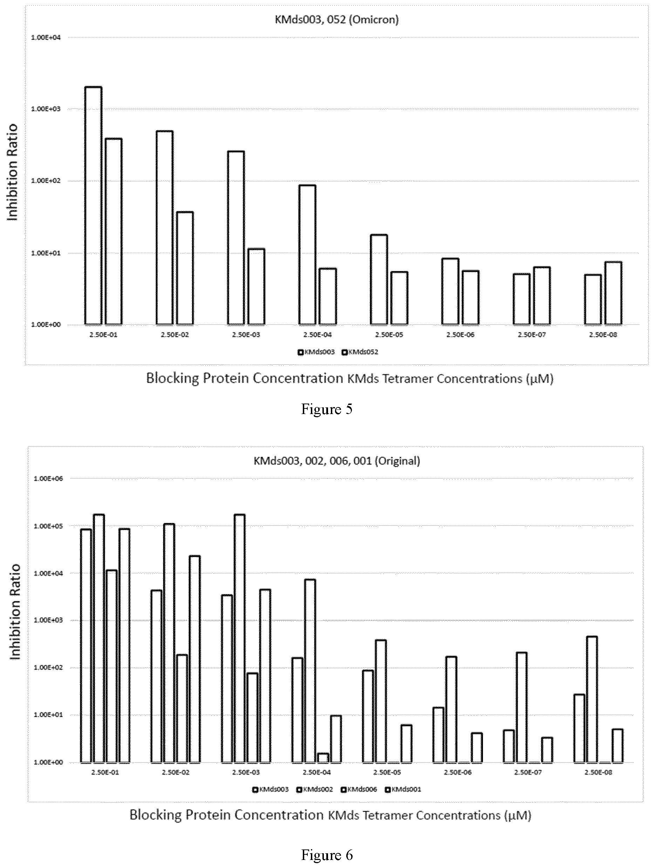

(2) As shown in FIG. 5, when the polymerizing molecule and the linker molecule was removed from 0052, 0052 at a relatively low concentration still has an inhibition effect.

III. Addition of acidic structure:

(1) The effect with adding an acidic structure is better than that without adding an acidic structure. As shown in FIG. 6, Kmds003, Kmds002, and Kmds006, into which an acidic structure was added respectively, have a better effect than Kmds001 without adding an acidic structure.

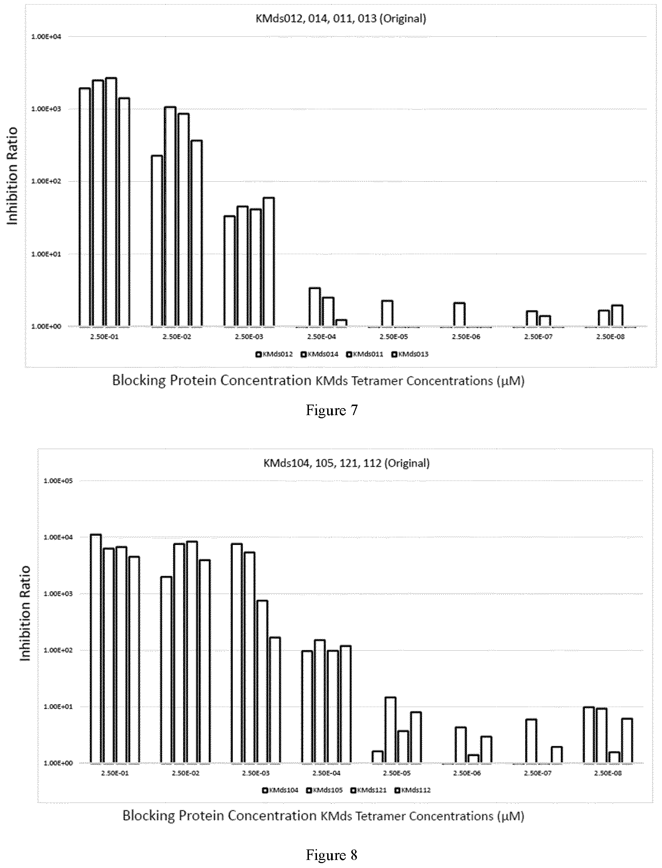

(2) The effect is better when 8d8e or 10e was added. As shown in FIG. 6, Kmds006 has a better effect of blocking virus than Kmds003 and Kmds002. As shown in FIG. 7, Kmds012 and Kmds014 have better effects than Kmds011 and Kmds013.

IV. Linker molecule:

(1) As shown in FIG. 8, the effects are similar when different linker molecules were used: KMds104, KMds105, KMds121, and KMds112.

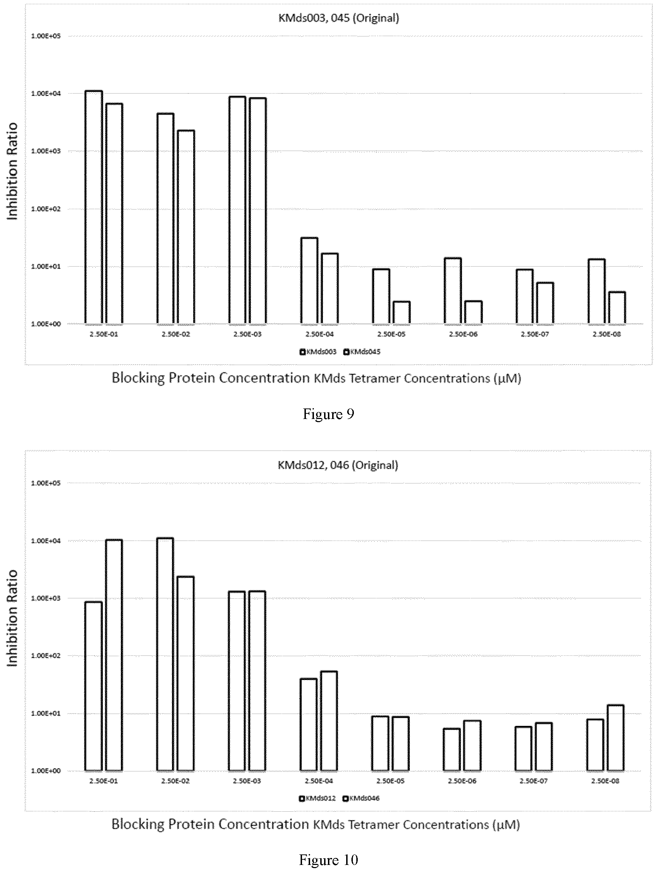

(2) eGFP was partially engineered and compared with KMds003, KMds012, KMds104 which was not engineered: After the engineering, KMds045, KMds046 and KMds109 were obtained respectively. The results are shown in FIGs. 9 to 11. The effects before and after the engineering are similar.

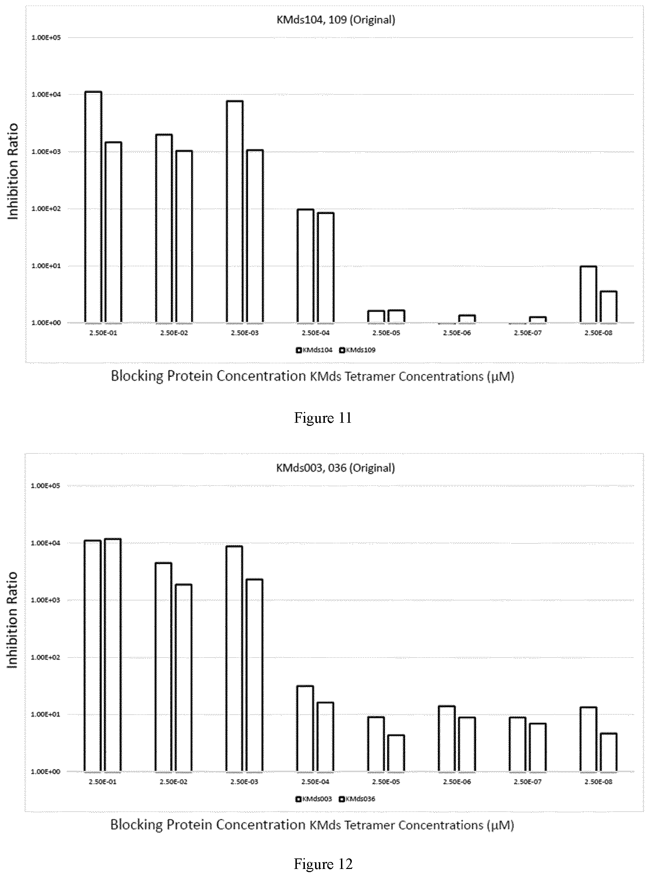

(3) After the linker molecule was deleted, the virus could still be suppressed and the results are shown in FIGs.12 to 14. KMds036, KMds038, and KMds108 were obtained by deleting Tram from KMds003, KMds012, and KMds104, respectively.

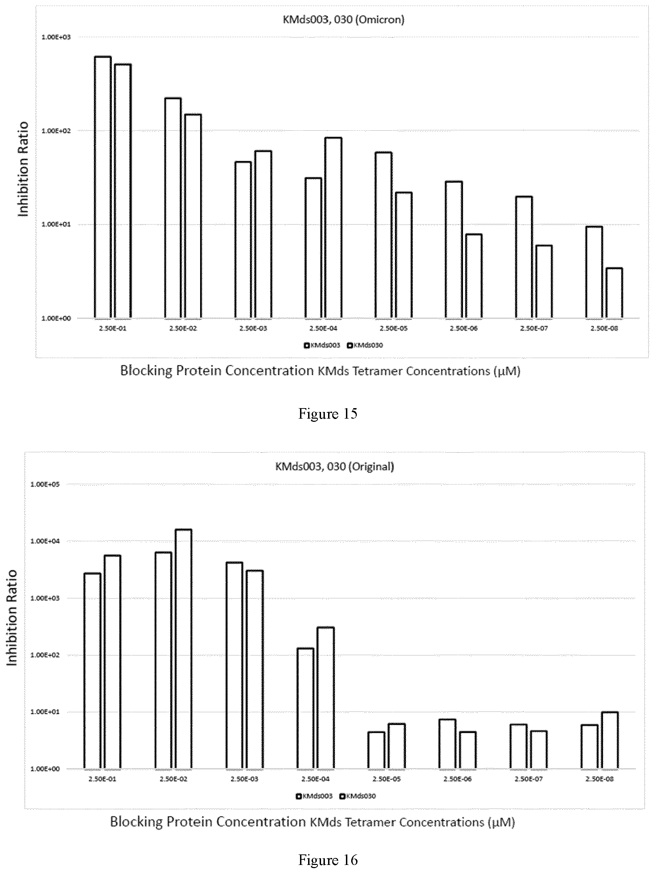

V. Leading peptide:
KMds030, KMds031, KMds032, KMds033, and KMds104 were obtained by deleting a leading peptide from KMds003, KMds006, KMds012, KMds014, and KMds044, respectively, and the results are shown in FIGs. 15 to 22. According to the results, the leading peptide has no effect on the virus inhibition of the virus-blocking protein.

VI. Cross-linking:
An electron microscope observation was carried out before and after virus was treated with the structures that have a polymerizing molecule in virus blocking test. As shown in FIG. 23, A in FIG. 23 is an image without treating with a virus-blocking protein, and others are other images that have different magnification after treating with the virus-blocking protein. As can be seen from the image, large spot was displayed in the image by the electron microscope after the virus was treated with the polymerizing molecule. That is, our structure enables cross-linking and aggregation of the virus, thereby improving cross-linking, blocking and inhibition on the virus.

VII. $IC_{50}$ results of Pseudovirus:
The test results show that in all tests, for the same blocking molecule, the lowest $IC_{50}$ value can be 42.4 pM (at the level of picomole) when it has an acidic structure, a polymerizing molecule, and a linker molecule; the $IC_{50}$ value can be about 0.222 nM when it has no acidic structure; the inhibition on pseudovirus will be reduced when the linker molecule, the polymerizing molecule, or both are not comprised, and $IC_{50}$ value can be 1.054, 2.404, and 1.032 nM, respectively.

5. Live virus test

We investigated the inhibition of the viral-blocking proteins in Table 2 against different SARS-CoV-2 strains, including the original strain, Alpha, Beta, Delta, and Omicron variants. The results are as follows:
The original strain was strongly inhibited by the virus-blocking proteins ($IC_{50}$ is 108.6 pM).
On the other hand, the viral-blocking proteins at very low concentrations can show effective inhibition on Alpha, Beta, Delta and Omicron variants ($IC_{50}$ is 92.8, 121.9, 61.0 and 121.9 pM, respectively).
The inhibition of the viral-blocking proteins on Delta variant is increased by approximately 1.8 folds compared with that on neutralizing the original SARS-CoV-2, and is almost the same on inhibiting other strains.
Tests on live viruses have shown that the viral-blocking proteins of the present disclosure still strongly inhibit different SARS-CoV-2 variants at pmol level, despite a large number of mutations in the spike protein of the variants.

6. Animal experiment

The SARS-CoV-2 wild-type (WT) strain (IVCAS 6.7512) was provided by the National Virus Resource Center of Wuhan Institute of Virology, Chinese Academy of Sciences. Heterozygous B6/JGpt-H11em1Cin(K18-ACE2)/Gpt mice (K18-hACE2 KI mice) were purchased from Nanjing GemPharmatech.

In a specific pathogen-free (SPF) environment, mice were housed and propagated in individually ventilated cages (IVCs). Animal experiments were carried out by the certified personnel of the Animal Experiment Center of Wuhan University and approved by the Institutional Animal Care and Use Committee (AUP #WP2021-0602). The protocols and procedures for infectious SARS-CoV-2 virus under the Animal Biosafety Level 3 Laboratory Facility had been approved by the Institutional Biosafety Committee (IBC, Protocol #S01322010A).

All samples were inactivated in accordance with the standard procedures approved by the IBC to remove the samples from the high seal. Each mouse was infected with $2.5 \times 10^2$ PFU SARS-CoV-2. For the negative control group, the SARS-CoV-2 was premixed with the control buffer for 30 minutes.

For the 0.25 nM virus-blocking protein premixing treatment group, SARS-CoV-2 was premixed with 0.25 nM virus-blocking protein for 30 minutes.

For the 25 nM virus-blocking protein premixing treatment group, SARS-CoV-2 was premixed with 25 nM virus-blocking protein for 30 minutes.

The K18-hACE2 mice were then intranasally inoculated with the premix.

The tissue was weighed and homogenized in 1000 $\mu$L PBS in the Tissue Celldestroyer 1000 instrument (NZK LTD). The tissue homogenate was clarified by centrifuging at 5, 000 rpm for 40 seconds, and 100 $\mu$L supernatant was mixed with 400 $\mu$L Trizol LS to extract viral RNA.

The pCMV-N plasmid and SARS-CoV-2 N gene primer (Forward primer: ATGCTGCAATCGTGCTACAA; and Reverse primer: GACTGCCGCCTCTGCTC) were used to construct the SARS-CoV-2 N gene standard curve and the virus copies were calculated. Data was analyzed by using Prism version 7 (GraphPad software) according to student test. P < 0.05 is considered to be statistically significant.

To test the role of virus-blocking proteins in mouse models, we used a premix of SARS-CoV-2 and virus-blocking protein to infect the K18-hACE2 mice. Mice were sacrificed on days 2 and 5 after infection (dpi). During the 5-day period, mice were 326 monitored daily for body weight changes and mortality rate.

K18-hACE2 mice intranasally inoculated with SARS-CoV-2 began to lose weight 3 to 4 days after infection and died at 4 dpi;

Mice inoculated with the premix of viral-blocking protein and SARS-CoV-2 significantly reduced weight loss and significantly increased survival compared to control mice, especially with high dosage viral-blocking protein.

High levels of SARS-CoV-2 RNA were detected in the lung tissue of K18-hACE2 infected with SARS-CoV-2, while low levels of viral RNA were detected in mice infected with the premix of SARS-CoV-2 and low dosage viral-blocking protein, and the viral RNA was even undetectable in lung tissue of mice infected with high dosage viral block protein and SARS-CoV-2 premix. In conclusion, these data indicated that high dosage viral-blocking protein can significantly reduce weight loss, improve survival in mice, and inhibit viral replication in mice.

7. Toxicity test

Toxicology test was carried out according to 2.3.1 Acute Oral Toxicity Test, Standard of Inspection Technology for Disinfection Product (Part 2), Disinfection Technical Standard (2002 edition). Abnormal toxicity test was carried out according to those recited in Principles and Inspection Methods for Biological Products, General Principles, Pharmacopoeia of the People's Republic of China, 2020 (No. IV).

ICR mice were provided by the Animal Center of Nanjing Medical University.

The Sprague Dawley (SD) rats were purchased from Pizhou Orient Breeding Co., Ltd.

The New Zealand White Rabbits were provided by Yizheng ANLIMAO Biotechnology Co., Ltd. The animals were housed at 20°C to 26°C and in a relative humidity of 40% to 70% in a local barrier system.

For the structures involved in Table 2, the following toxicity tests were carried out:

(1) Acute oral toxicity

Acute Oral Toxicity Test was carried out on twenty SPF ICR mice (18.0 to 22.0 g) and twenty SPF SD rats (180~220 g). The number of males was equal to that of females. Mice and rats fasted overnight were treated with the virus-blocking proteins prepared according to the formulas in Table 3, at a dosage of 5000 mg/kg•bw, by gastric intubation for a single dose. The animals were then monitored weekly for clinical signs of toxicity and mortality rate for 14 days (on Days 0, 7, and 14). Behavior, number of deaths and body weight were evaluated, respectively, and the animals were dissected at the end of the observation period.

According to the results of the test, the virus-blocking proteins provided at a dosage of 5000 mg/kg•bw led to normal weight and no onset in mice and rats fasted overnight. The LD50 values of virus-blocking proteins against mice and rats were 5000 mg/kg•bw or more. No signs of toxicity and death were found during the observation.

(2) Acute inhalation toxicity test

Acute inhalation toxicity test was carried out on twenty SPF ICR mice (18.0~22.0 g). The number of males was equal to that of females. 2.2 g of the virus-blocking proteins prepared corresponding to Table 3 were placed in 220 L of toxicant exposure cabinet to a assumed concentration of 10,000 mg/m$^3$. The inhalation exposure time was set to 2 hours. Symptoms and deaths in rats were recorded during the 14-day observation period (on Days 0, 7, and 14).

According to the test report, the LC50 values of the virus-blocking protein after 2-hour exposure were greater than 10,000 mg/m$^3$ in both female and male mice. The mice showed normal and stable weight gain and no abnormal signs. Therefore, under current experimental conditions, the viral-blocking proteins are considered non-toxic and compliant to provision.

(3) Acute eye irritation test

Acute eye irritation test was carried out on three male New Zealand rabbits (2.5 to 3.5 kg). 0.1 mL of the virus-blocking protein stock solution prepared in Table 3 was dropped into the conjunctiva sacs of the right eyes of the rabbits, and normal saline as a control was dropped into the left eye. 30 seconds later after closing the eyes for 4 seconds, the eyes were rinsed with normal saline. The injury and recovery of conjunctiva, iris and cornea of rabbits were observed for 21 days (at 1 hour, 24 hours, 48 hours, 72 hours, on days 7, 14 and 28). The severity of corneal injury, iris injury, conjunctival congestion, and conjunctival edema were scored.

According to the test results, the tested virus-blocking proteins showed no signs of eye irritation in rabbits. The scores of the three rabbits at 24 hours, 48 hours and 72 hours were less than 1, as shown in Table 1. Thus, the irritation of the virus-blocking proteins of the present disclosure is classified as non-irritation.

(4) Micronucleus test in mouse bone marrow polychromatic erythrocytes (PCEs)

Micronucleus test in mouse bone marrow polychromatic erythrocytes was carried out on fifty ICR mice. The number of males was equal to that of females. The animals were divided into five groups, with five female mice and five male mice in each group. The test group were administered once with the virus-blocking proteins prepared in Table 3 at a dosage of 5000, 2500 and 1250 mg/kg•bw, respectively. One group as a negative control was treated with purified water as a solvent. An anther group as a positive control was administered intraperitoneally with 40 mg/kg•bw cyclophosphamide (CP). The test group was exposed to the virus-blocking protein by oral administration at 0 and 24 hours. At six hours after the second exposure to the virus blocking protein, mice were sacrificed to prepare bone marrow smears. Micronuclei were calculated in 1000 polychromatic erythrocytes (PCEs) per animal. Once 200 PCEs were counted, the ratio of PCE to NCE was determined. Statistical analysis was performed by U test. When the incidence of micronucleus formation was significantly increased in the test group compared with the negative control, it was confirmed that the test drug was harmful to chromosomes in vivo, which would occur in according to a dose-response relationship.

For the micronucleus test, the micronucleus formation rate in female and male mice was not significantly different in the three groups compared with the negative group ($P < 0.05$), but significantly different from the positive control group ($P < 0.05$), as shown in Table 2. In addition, the PCE/NCE ratio between any test group and negative control group varies within 20%.

(5) Abnormal toxicity test

Abnormal toxicity test was carried out on ten female SPF ICR mice (18.0 to 22.0 g) and four standard grade female guinea pigs (250 to 350 g).

Ten mice were randomly divided into a test group and a control group with 5 mice in each group. The test group was injected intraperitoneally with 0.5 mL of the sample and the control group was injected intraperitoneally with 0.5 mL of sodium chloride. The two groups were observed for their physique for 7 days. Four female guinea pigs were randomly divided into the test group and the control group with 2 guinea pigs in each group. The test group was injected intraperitoneally with 0.5 mL of sample and the control group was injected intraperitoneally with 0.5 mL of sodium chloride. The two groups were observed for their physique for 7 days.

In all tests, the virus-blocking proteins provided in the present disclosure did not cause observable toxic effect.

8. Test of the thermal stability of the virus blocking protein.

The thermal stability of several proteins was tested using the Uncle equipment from Unchained. Tmagg represents the temperature at which the protein is highly polymerized during heating. The results are shown in Table 5.

| Table 5 | |
|---|---|
| Sample | Average Tmagg 266 (°C) |
| 0.5 mg/ml KM001 | 48.4 |
| 0.5 mg/ml KM003 | 53.2 |
| 0.5 mg/ml KM007 | 82.6 |
| 0.5 mg/ml KM011 | 83.5 |
| 0.5 mg/ml KM012 | 84.3 |
| 0.5 mg/ml KM013 | 84.2 |
| 0.5 mg/ml KM006 | 88.9 |
| 0.5 mg/ml KM008 | 86.3 |
| 0.5 mg/ml KM010 | 45.0 |
| 0.5 mg/ml KM009 | 42.2 |

Meanwhile, Tmagg value for Kmds001 containing B 1 and B2 is less than 50°C, but Kmds006 with adding the acidic structure 8D8E has a much higher Tmagg value than, and nearly twice as high as, Kmds001 where no tail is added. That is, this acidic structure can increase the stability of the protein.

In addition, the average Tm of the viral-blocking proteins have the respective structures in Table 2 was tested to be close to 80°C, indicating that the viral-blocking proteins provided in the present disclosure can be protected from an extreme temperature.

9. Accelerated stability test

Stability studies were carried out at two different temperatures, both 4°C and 37°C (in triplicate, 100 uL of 10 nM virus-blocking protein).

The other group in which no virus-blocking protein was added was used as a negative control (NC). Sampling was carried out thrice every 15 days for 90 days (on days 0, 15, 30, 45, 60, 75 and 90). The percent inhibition is evaluated by the pseudovirus neutralization test and calculated according to the following formula: Percent inhibition = (NC - sample)/NC (in RFU).

The test results showed that, after storage for more than 90 days at 4°C or 37°C, the inhibition efficiency of the viral-blocking proteins was almost unchanged (> 99.9%), indicating that the viral-blocking proteins of the present disclosure is an ultra-stable SARS-CoV-2 blocker.

The following example is an example for films. The virus-blocking protein involved in the example was the virus-blocking protein prepared by referring to each structure involved in the method in example 1. The stock solution of the virus-blocking protein used in each formulation has a concentration of about 2 mg/ml.

The experimental hygroscopic method in the following example is as follows:

Equipment: Humidifier; Model: KW-AD01; Power: 5W; and Capacity: 100 ml.

The humidifier was filled with water, the prepared films were placed at 5 to 10 centimeters over the atomizing nozzle of the humidifier, and the moisture absorption change of the films was observed. The experiment was carried out at room temperature.

10. Test of the binding capability of ammonium sulfate-precipitated IVTT reaction solution of a structure provided in Table 3.

After IVTT reaction, the solution was precipitated with ammonium sulfate and centrifuged at 4000 rpm, 4°C, for 10 minutes. 5% (w/v) ammonium sulfate powder was added to the supernatant, fully stirred for dissolution, and centrifuged at 4000 rpm, 4°C, for 10 minutes. 25% (w/v) ammonium sulfate powder was added slowly again to the supernatant and stirred for dissolution. The resulted solution was centrifuged at 12,000 rpm, 4°C, for 10 minutes. The supernatant was discarded. The precipitate was rinsed once with 30% (w/v) ammonium sulfate solution, and centrifuged at 12,000 rpm, 4°C, for 10 minutes. The precipitate was resuspended in PBS for thorough dissolution, centrifuged at 12,000 rpm, 4°C, for 10 minutes, filtered through a 0.22 $\mu$m syringe filter, and saved at 4°C for later use.

The sample activity of ammonium sulfate-precipitated IVTT reaction solution of Kmds012 is slightly lower than that of the Kmd012 purified protein (i.e. bead-purified protein).

It can be seen that the polymer structure provided in the present disclosure can be further purified by simple and convenient ammonium sulfate precipitation.

**Claims**

1.  A polymerizing molecule, which is configured to polymerizing a plurality of binding-blocking molecular units that block binding between a virus and a cell receptor into a polymer structure.

2.  The polymerizing molecule according to claim 1, wherein the polymerizing molecule has a monomer binding site and a polymerizing site,

    the monomer binding site is configured to bind to a binding-blocking molecular unit so as to form a monomer structure having at least the polymerizing molecule and the binding-blocking molecular unit, and a polymer structure is formed by polymerizing a plurality of the monomer structures through the polymerizing site; and preferably, the polymerizing molecule is a peptide or a protein, the monomer binding site is located at an N-terminus of the polymerizing molecule.

3.  The polymerizing molecule according to claim 1 or 2, wherein the polymer structure is formed by polymerizing 2 to 10 monomer structures.

4.  The polymerizing molecule according to any one of claims 1 to 3, wherein the polymerizing molecule is any one selected from Table 1.

5.  The polymerizing molecule according to claim 4, wherein the polymerizing molecule is streptavidin, preferably, streptavidin comprises an amino acid sequence that is identical to SEQ ID NO: 1 or has at least 50%, 60%, 70, 80%, 85%, 90%, 95% or 99% identity to SEQ ID NO: 1.

6. The polymerizing molecule according to claim 6, wherein the polymerizing molecule binds to the binding-blocking molecule unit via a linker molecule;

> the linker molecule comprises any one or more of a fluorescent protein, a human immunoglobulin G4, a Fc, and an HAS; for example, the linker molecule is fluorescent protein eGFP or is obtained by means of modification of the fluorescent protein eGFP, preferably by deleting some amino acids from eGFP; or
> the linker molecule comprises an amino acid sequence that is identical to any one of SEQ ID NOs: 2 to 6 or has at least 50%, 60%, 70, 80%, 85%, 90%, 95% or 99% identity to any one of SEQ ID NOs: 2 to 6, preferably obtained by deleting the amino acids at positions 1 to 228 from eGFP;
> preferably, the linker molecule has an N-terminus that is linked to the binding-blocking molecule unit, and a C-terminus that is linked to the N-terminus of the polymerizing molecule.

7. The polymerizing molecule according to any one of claims 1 to 6, wherein the binding-blocking molecule unit blocks the binding between the virus and a cell receptor by binding to a site of the virus at which the virus binds to the cell receptor, and/or the binding-blocking molecule unit blocks the binding between the virus and the cell receptor by binding to the cell receptor.

8. The polymerizing molecule according to any one of claims 1 to 7, wherein the binding-blocking molecule unit comprises at least one blocking molecule that blocks binding between a virus and a cell receptor;

> preferably, the blocking molecule comprises an amino acid sequence that is identical to any one of SEQ ID NOs: 7 to 9 and 16 to 19 or has at least 50%, 60%, 70, 80%, 85%, 90%, 95% or 99% identity to any one of SEQ ID NOs: 7 to 9 and 16 to 19; further, when the binding-blocking molecule unit comprises a plurality of blocking molecules, the blocking molecules are linked sequentially to each other in a direction from the N-terminus to the C-terminus; more preferably, when the binding-blocking molecule unit is linked to the polymerizing molecule via the linker molecule, the N-terminus of the linker molecule is linked to the C-terminus of the last blocking molecule, and the C-terminus of the linker molecule is linked to the monomer binding site; and
> still more preferably, the blocking molecule is a nanobody.

9. The polymerizing molecule according to any one of claims 1 to 8, wherein the cell receptor is ACE2.

10. The polymerizing molecule according to claim 8 or 9, wherein the binding-blocking molecule unit comprises at least one first blocking molecule and/or at least one second blocking molecule; the first blocking molecule comprises an amino acid sequence that is identical to SEQ ID NO: 7 or has at least 50%, 60%, 70, 80%, 85%, 90%, 95% or 99% identity to SEQ ID NO: 7, and the second blocking molecule comprises an amino acid sequence that is identical to any one of SEQ ID NOs: 8 and 16 to 19 or has at least 50%, 60%, 70, 80%, 85%, 90%, 95% or 99% identity to any one of SEQ ID NOs: 8 and 16 to 19;

> preferably, the binding-blocking molecule unit comprises two first blocking molecules or two second blocking molecules, and more preferably, the two first blocking molecules or the two second blocking molecules are linked to each other respectively via a N-terminus of one to a C-terminus of the other;
> still more preferably, the binding-blocking molecule unit comprises the first blocking molecule and the second blocking molecule, of which the N-terminus of one is linked to the C-terminus of the other, and more preferably, the N-terminus of the second blocking molecule is linked to the C-terminus of the first blocking molecule.

11. The polymerizing molecule according to any one of claims 1 to 10, wherein the monomer structure further comprises a leading peptide,

> the leading peptide comprises an amino acid sequence that is identical to SEQ ID NO: 10 or has at least 50%, 60%, 70, 80%, 85%, 90%, 95% or 99% identity to SEQ ID NO: 10; and
> preferably, the leading peptide has a C-terminus that is linked to the N-terminus of any one of the blocking peptide fragments.

12. The polymerizing molecule according to any one of claims 1 to 11, wherein the monomer structure further comprises an acidic structure; the acidic structure is a short-chain polymer of amino acids which is negatively charged, and further, the acidic structure has one or more of the following characteristics:

> (1) the acidic structure is located at the C-terminus;

(2) the short-chain polymer has 0 to 50, 2 to 40, 3 to 30, 2 to 20, or 2 to 10 amino acid residues; and

(3) the negatively charged amino acids are aspartate and/or glutamate,

preferably, the acidic structure is linked to the C-terminus of the polymerizing molecule.

13. The polymerizing molecule according to any one of claims 1 to 12, wherein the monomer structure further comprises a protein tag;

the protein tag comprises an amino acid sequence that is identical to SEQ ID NO: 15 or has at least 50%, 60%, 70, 80%, 85%, 90%, 95% or 99% identity to SEQ ID NO: 15,

preferably, the protein tag has a C-terminal that is linked to the N-terminal of a binding-blocking molecule unit, and when the binding-blocking molecule unit is blocking peptide fragments, the C-terminal of the protein tag is linked to the N-terminal of any one of blocking peptide fragments; and

more preferably, when the monomer structure further comprises the leading peptide, the leading peptide is linked to the binding-blocking molecule unit via the protein tag.

14. The polymerizing molecule according to any one of claims 1 to 13, wherein the virus is one or more of hepatitis B virus, rabies virus, HVP, and COVID-19 virus.

15. A monomer structure comprising the polymerizing molecule according to any one of claims 1 to 14,

preferably, the monomer structure has a size of 30 to 80 KD;

still more preferably, the monomer structure is a monomer structure according to any one of claims 2 to 14.

16. A polymer structure formed by polymerizing a plurality of the monomer structures according to claim 15.

17. The polymer structure according to claim 16, wherein the polymer structure is formed by polymerizing 2 to 10 monomer structures, and

preferably, the polymer structure is formed by polymerizing four monomer structures.

18. The polymer structure according to claim 16 or 17, wherein the blocking structure unit has a binding force that is 1000 to 1000,000 times greater than a nanobody.

19. The polymer structure according to any one of claims 16 to 18, wherein the polymer structure is soluble.

20. A nucleic acid encoding the polymerizing molecule according to any one of claims 1 to 15, the monomer structure according to claim 15, or the polymer structure according to any one of claims 16-19.

21. A vector comprising the nucleic acid according to claim 20.

22. A eukaryotic host cell for the expression in cells, secretion from cells or in vitro cell-free synthesis and expression of the monomer structure according to claim 15 or the polymer structure according to any one of claims 16 to 19, comprising the nucleic acid according to claim 20 or the vector according to claim 21.

23. Use of the nucleic acid according to claim 20 or the vector according to claim 21 in a method for preparing the monomer structure according to claim 15 and/or the polymer structure according to any one of claims 16 to 19.

24. Use of any one of the polymerizing molecule according to any one of claims 1 to 14, the monomer structure according to claim 15, and the polymer structure according to any one of claims 16 to 19 in: a medicament for treating a virus, detection and diagnosis of a virus, a medical use, a disinfection product against a virus, a cosmetic product, a skin care product, a care product, a food, or a cleaning product,

preferably, an unpurified product obtained by in vitro cell-free synthesis of the polymer structure is directly applied for the use.

25. A disinfection product, a cosmetic product, a cosmetic product, a skin care product, a care product, a food or a cleaning product comprising one or more of any one of the polymerizing molecule according to any one of claims 1 to 14, the monomer structure according to claim 15, and the polymer structure according to any one of claims 16 to 19.

26. A medicament comprising: one or more of the polymerizing molecule according to any one of claims 1 to 14, the monomer structure according to claim 15, and the polymer structure according to any one of claims 16 to 19; and a pharmaceutically acceptable carrier, diluent, or excipient.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

Figure 19

Figure 20

Figure 21

Figure 22

Figure 23

Figure 24

Figure 25

## INTERNATIONAL SEARCH REPORT

| | |
|---|---|
| | International application No. |
| | **PCT/CN2023/070015** |

| | |
|---|---|
| **A.** | **CLASSIFICATION OF SUBJECT MATTER** |

C07K19/00(2006.01)i;C12N15/00(2006.01)i;A61P31/00(2006.01)i

According to International Patent Classification (IPC) or to both national classification and IPC

| | |
|---|---|
| **B.** | **FIELDS SEARCHED** |

Minimum documentation searched (classification system followed by classification symbols)

C07K、C12N、A61P

Documentation searched other than minimum documentation to the extent that such documents are included in the fields searched

Electronic data base consulted during the international search (name of data base and, where practicable, search terms used)

Databases: DWPI, SIPOABS, CNABS, ISI Web of knowledge, EMBASE , NCBI PUBMED, CNKI, MEDLINE, Google Scholar, GenBank, EMBL, 中国专利生物序列检索系统, China Patent Biological Sequence Search System; Key words: 病毒, 受体, 肽, 蛋白, 链霉素, 亲和素, 聚合, 多聚, 融合, virus, SARS, COVID, receptor, Multimeric , polymer, fuse, tamavidin, streptavidin, SA, peptide, protein, pep50, pep160, octaTag, 对SEQ ID NO.1, 2的检索, search for SEQ ID NO.1, 2

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| A | CN 101094688 A (GENZYME CORPORATION) 26 December 2007 (2007-12-26) claims | 1-11, 13-23, 25, 26 |
| A | CN 107592865 A (JANSSEN VACCINES & PREVENTION B.V.) 16 January 2018 (2018-01-16) claims | 1-11, 13-23, 25, 26 |
| A | CN 1798770 A (UNIVERSITY DE LAUSANNE) 05 July 2006 (2006-07-05) claims | 1-11, 13-23, 25, 26 |
| A | WO 2021219121 A1 (YANG SHENG TANG CO., LTD.; XIAMEN UNIVERSITY) 04 November 2021 (2021-11-04) claims | 1-11, 13-23, 25, 26 |

| | |
|---|---|
| ☑ Further documents are listed in the continuation of Box C. | ☑ See patent family annex. |

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **12 May 2023** | **25 May 2023** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2023/070015** |

**C.    DOCUMENTS CONSIDERED TO BE RELEVANT**

| Category* | Citation of document, with indication, where appropriate, of the relevant passages | Relevant to claim No. |
|---|---|---|
| A | YAHI, N. et al. "Synthetic Multimeric Peptides Derived from the Principal Neutralization Domain (V3 Loop) of Human Immunodeficiency Virus Type 1 (HIV-1) gp120 Bind to Galactosylceramide and Block HIV-1 Infection in a Human CD4-Negative Mucosal Epithelial Cell Line"<br>*Journal of Virology*, Vol. 69, No. (1), 31 January 1995 (1995-01-31),<br>    abstract, figure 1 | 1-11, 13-23, 25, 26 |

Form PCT/ISA/210 (second sheet) (July 2022)

## INTERNATIONAL SEARCH REPORT

| International application No. |
|---|
| **PCT/CN2023/070015** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☐ forming part of the international application as filed.

    b. ☑ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

64

## INTERNATIONAL SEARCH REPORT

| International application No. |
| --- |
| **PCT/CN2023/070015** |

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **24**
   because they relate to subject matter not required to be searched by this Authority, namely:

   The subject matter of claim 24 relates to a method for treatment of the human or animal body (medical use), which falls within the cases set out in PCT Rule 39.1(iv) for which no search is required.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

There are eight options for the polymerized structural unit of a polymeric molecule in the present application, and by combining the number of units, the types of other components and the connection sequence, a large number of technical solutions relating to different polymeric molecules can be obtained, and the total number of technical solutions exceeds 1000, comprising:

(1) in claims 1-11, 13-23, 25 and 26, the technical solutions relating to the Kmds001 structure in table 2;

(2) in claims 1-23, 25 and 26, the technical solutions relating to the Kmds002 structure in table 2;

(3) in claims 1-23, 25 and 26, the technical solutions relating to the Kmds003 structure in table 2;

......

The same feature of the above-mentioned groups of technical solutions is merely "forming multimers by a fused protein comprising a part blocking a virus from binding to a receptor and a polymerization part". However, the technical solutions of such kind of fused protein/multimer are disclosed in the prior art, for example, WO 2021219121 A1, publication date being 04 November 2021. The above inventions do not have the same or corresponding technical feature, do not have the same or corresponding special technical feature, do not belong to a single general inventive concept, and therefore lack unity of invention and do not comply with PCT Rule 13.1, 13.2 and 13.3.

Form PCT/ISA/210 (continuation of first sheet) (July 2022)

## INTERNATIONAL SEARCH REPORT

| International application No. |
| --- |
| **PCT/CN2023/070015** |

**Box No. III      Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Claims 1-11, 13-23, 25 and 26 (in part): the technical solutions relating to the Kmds001 structure in table 2**

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
Information on patent family members

| | | | International application No.<br>**PCT/CN2023/070015** |
|---|---|---|---|

| Patent document<br>cited in search report | Publication date<br>(day/month/year) | Patent family member(s) | | | Publication date<br>(day/month/year) |
|---|---|---|---|---|---|
| CN 101094688 A | 26 December 2007 | US | 2007224178 | A1 | 27 September 2007 |
| | | US | 7928072 | B2 | 19 April 2011 |
| | | JP | 2008512127 | A | 24 April 2008 |
| | | JP | 4944032 | B2 | 30 May 2012 |
| | | IL | 181839 | A0 | 04 July 2007 |
| | | PT | 1804835 | E | 04 October 2010 |
| | | BRPI | 0515264 | A | 15 July 2008 |
| | | BRPI | 0515264 | B1 | 18 December 2018 |
| | | US | 2011268735 | A1 | 03 November 2011 |
| | | US | 8658602 | B2 | 25 February 2014 |
| | | DK | 1804835 | T3 | 11 October 2010 |
| | | PL | 1804835 | T3 | 30 November 2010 |
| | | PL | 2229956 | T3 | 30 September 2013 |
| | | DK | 2229956 | T3 | 29 July 2013 |
| | | WO | 2006031689 | A2 | 23 March 2006 |
| | | WO | 2006031689 | A3 | 06 July 2006 |
| | | MX | 2007002942 | A | 05 March 2008 |
| | | IL | 216095 | A0 | 29 December 2011 |
| | | IL | 216095 | A | 31 October 2013 |
| | | HK | 1144663 | A1 | 04 March 2011 |
| | | ES | 2407859 | T3 | 14 June 2013 |
| | | US | 2018155417 | A1 | 07 June 2018 |
| | | DE | 602005021811 | D1 | 22 July 2010 |
| | | ES | 2347340 | T3 | 28 October 2010 |
| | | US | 2014193411 | A1 | 10 July 2014 |
| | | US | 9815892 | B2 | 14 November 2017 |
| | | EP | 2229956 | A1 | 22 September 2010 |
| | | EP | 2229956 | B1 | 24 April 2013 |
| | | AT | 470454 | T | 15 June 2010 |
| | | EP | 1804835 | A2 | 11 July 2007 |
| | | EP | 1804835 | A4 | 30 July 2008 |
| | | EP | 1804835 | B1 | 09 June 2010 |
| | | EP | 1804835 | B9 | 03 November 2010 |
| | | PT | 2229956 | E | 31 July 2013 |
| | | CY | 1114155 | T1 | 31 August 2016 |
| CN 107592865 A | 16 January 2018 | PH | 12017501943 | A1 | 19 March 2018 |
| | | EP | 3294753 | A1 | 21 March 2018 |
| | | JP | 2018522819 | A | 16 August 2018 |
| | | JP | 6921002 | B2 | 18 August 2021 |
| | | BR | 112017024206 | A2 | 17 July 2018 |
| | | IL | 255542 | A | 31 January 2018 |
| | | IL | 255542 | B | 01 June 2022 |
| | | AU | 2016261299 | A1 | 23 November 2017 |
| | | AU | 2016261299 | A9 | 25 July 2019 |
| | | AU | 2016261299 | B2 | 05 December 2019 |
| | | CA | 2985402 | A1 | 17 November 2016 |
| | | KR | 20180002713 | A | 08 January 2018 |
| | | EA | 201792482 | A1 | 28 February 2018 |
| | | MX | 2017014343 | A | 23 March 2018 |
| | | US | 2018118788 | A1 | 03 May 2018 |

Form PCT/ISA/210 (patent family annex) (July 2022)

**INTERNATIONAL SEARCH REPORT**
Information on patent family members

International application No.

**PCT/CN2023/070015**

| Patent document cited in search report | | | Publication date (day/month/year) | Patent family member(s) | | | Publication date (day/month/year) |
|---|---|---|---|---|---|---|---|
| | | | | US | 10738083 | B2 | 11 August 2020 |
| | | | | WO | 2016180826 | A1 | 17 November 2016 |
| | | | | WO | 2016180826 | A8 | 01 February 2018 |
| CN | 1798770 | A | 05 July 2006 | US | 2006233808 | A1 | 19 October 2006 |
| | | | | NO | 20055209 | D0 | 04 November 2005 |
| | | | | NO | 20055209 | L | 02 January 2006 |
| | | | | KR | 20060017585 | A | 24 February 2006 |
| | | | | WO | 2004087766 | A2 | 14 October 2004 |
| | | | | WO | 2004087766 | A3 | 16 December 2004 |
| | | | | AT | 514718 | T | 15 July 2011 |
| | | | | AU | 2004226162 | A1 | 14 October 2004 |
| | | | | EP | 1613661 | A2 | 11 January 2006 |
| | | | | EP | 1613661 | B1 | 29 June 2011 |
| | | | | JP | 2007527206 | A | 27 September 2007 |
| | | | | CA | 2521393 | A1 | 14 October 2004 |
| | | | | BRPI | 0409554 | A | 25 April 2006 |
| | | | | MXPA | 05010575 | A | 09 March 2006 |
| WO | 2021219121 | A1 | 04 November 2021 | EP | 4144765 | A1 | 08 March 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 108690139 A
- CN 109423496 A
- CN 106978439 A
- CN 110408635 A
- CN 110551700 A
- CN 110093284 A
- CN 110845622 A
- CN 110938649 A
- CN 111378708 A
- CN 111484998 A
- CN 106978349 A
- CN 108535489 A
- CN 108949801 A
- CN 108642076 A
- CN 109022478 A
- CN 109423497 A
- CN 109423509 A
- CN 109837293 A
- CN 109971783 A
- CN 109988801 A
- CN 109971775 A
- CN 110408636 A
- CN 110551745 A
- CN 110551785 A
- CN 110819647 A
- CN 110964736 A

### Non-patent literature cited in the description

- *Molecular andCellular Biology,* 1990, vol. 10 (1), 353-360